# EUROPEAN PATENT SPECIFICATION

(11) **EP 3 101 035 B1**
(45) Date of publication and mention of the grant of the patent: **06.10.2021**
(21) Application number: 15742538.0
(22) Date of filing: 28.01.2015
(51) Int. Cl.: C07K 19/00, C07K 16/24, C07K 14/705, C12N 15/62, C12N 15/63, C12N 5/10, A61K 38/17, A61K 39/395, A61P 37/02

(54) **BIFUNCTIONAL FUSION PROTEIN, PREPARATION METHOD THEREFOR, AND USE THEREOF**
BIFUNKTIONELLES FUSIONSPROTEIN, HERSTELLUNGSVERFAHREN DAFÜR UND VERWENDUNG DAVON
PROTÉINE DE FUSION BIFONCTIONNELLE, PROCÉDÉ DE PRÉPARATION S'Y RAPPORTANT ET SON UTILISATION

(30) Priority: 28.01.2014 CN 201410042808
(43) Date of publication of application: 07.12.2016
(73) Proprietor: Beijing Hanmi Pharmaceutical Co., Ltd., Beijing 101312 (CN)
(72) Inventor: CHEN, Guoqiang, Beijing 101312 (CN); LIU, Jiawang, Beijing 101312 (CN); SONG, Nanmeng, Beijing 101312 (CN); YANG, Yaping, Beijing 101312 (CN); CHA, Mi Young, Beijing 101312 (CN)
(74) Representative: Inspicos P/S
(86) International application number: PCT/CN2015/071718
(87) International publication number: WO 2015/113494

(56) References cited:
- WO-A1-2009/147362
- WO-A1-2009/147362
- WO-A1-2013/041029
- WO-A1-2013/041029
- WO-A1-2013/169338
- CN-A- 101 001 645
- DATABASE MEDLINE [Online] US NATIONAL LIBRARY OF MEDICINE (NLM), BETHESDA, MD, US; June 2012 (2012-06), KOENDERS MARIJE I ET AL: "T cell lessons from the rheumatoid arthritis synovium SCID mouse model: CD3-rich synovium lacks response to CTLA-4Ig but is successfully treated by interleukin-17 neutralization.", XP002770164, Database accession no. NLM22213107
- FIOCCO U ET AL: "Co-stimulatory modulation in rheumatoid arthritis: The role of (CTLA4-Ig) abatacept", AUTOIMMUNITY REVIEWS, ELSEVIER, AMSTERDAM, NL, vol. 8, no. 1, 1 October 2008 (2008-10-01) , pages 76-82, XP025505755, ISSN: 1568-9972, DOI: 10.1016/J.AUTREV.2008.07.035 [retrieved on 2008-08-15]
- NOGID A ET AL: "Role of abatacept in the management of rheumatoid arthritis", CLINICAL THERAPEUTICS, EXCERPTA MEDICA, PRINCETON, NJ, US, vol. 28, no. 11, 1 November 2006 (2006-11-01), pages 1764-1778, XP027906871, ISSN: 0149-2918 [retrieved on 2006-11-01]
- DESHAN LI: "Chimeric anti-IL-17 full-length monoclonal antibody is a novel potential candidate for the treatment of rheumatoid arthritis", INTERNATIONAL JOURNAL OF MOLECULAR MEDICINE, 27 December 2013 (2013-12-27), XP055372649, GR ISSN: 1107-3756, DOI: 10.3892/ijmm.2013.1611
- VUGMEYSTER YULIA ET AL: "Pharmacokinetics of anti-IL17A and anti-IL22 peptide-antibody bispecific genetic fusions in", INTERNATIONAL IMMUNOPHARMACOLOGY, ELSEVIER, AMSTERDAM, NL, vol. 18, no. 2, 2 December 2013 (2013-12-02), pages 225-227, XP028819170, ISSN: 1567-5769, DOI: 10.1016/J.INTIMP.2013.11.013
- MORELAND L W ET AL: "COSTIMULATORY BLOCKADE IN PATIENTS WITH RHEUMATOID ARTHRITIS", ARTHRITIS & RHEUMATISM, WILEY INTERSCIENCE, US, vol. 46, no. 6, 1 June 2002 (2002-06-01), pages 1470-1479, ISSN: 0004-3591, DOI: 10.1002/ART.10294
- PIERRE MIOSSEC ET AL: "Targeting IL-17 and TH17 cells in chronic inflammation", NATURE REVIEWS DRUG DISCOVERY, vol. 11, no. 10, 1 October 2012 (2012-10-01), pages 763-776, ISSN: 1474-1776, DOI: 10.1038/nrd3794
- KREMER JOEL M ET AL: "Treatment of rheumatoid arthritis with the selective costimulation modulator abatacept: twelve-month results of a phase iib, double-blind, randomized, placebo-controlled trial.", August 2005 (2005-08), ARTHRITIS AND RHEUMATISM AUG 2005, VOL. 52, NR. 8, PAGE(S) 2263 - 2271 ISSN: 0004-3591
- MIOSSEC PIERRE ET AL: "Interleukin-17 and type 17 helper T cells.", 27 August 2009 (2009-08-27), THE NEW ENGLAND JOURNAL OF MEDICINE 27 AUG 2009, VOL. 361, NR. 9, PAGE(S) 888 - 898 ISSN: 1533-4406
- BERINGER AUDREY ET AL: "IL-17 in Chronic Inflammation: From Discovery to Targeting", TRENDS IN MOLECULAR MEDICINE, ELSEVIER CURRENT TRENDS, GB, vol. 22, no. 3, 31 January 2016 (2016-01-31), pages 230-241, ISSN: 1471-4914, DOI: 10.1016/J.MOLMED.2016.01.001
- VAN DEN BERG WIM B ET AL: "Th17 cells and IL-17 A-Focus on immunopathogenesis and immunotherapeu", SEMINARS IN ARTHRITIS AND RHEUMATISM, vol. 43, no. 2, 2013, pages 158-170, ISSN: 0049-0172, DOI: 10.1016/J.SEMARTHRIT.2013.04.006

## Description

### FIELD OF THE INVENTION

The present invention relates to a bifunctional fusion protein, a preparation method therefor and use thereof. In particular, the present invention relates to a bifunctional recombinant fusion protein which can block B7/CD28 and IL-17A/IL-17RA signal pathways simultaneously, a gene encoding the bifunctional fusion protein, a vector comprising the gene, a host cell comprising the vector, and a pharmaceutical composition containing the bifunctional fusion protein.

### BACKGROUND

It is regarded as an effective way for treating chronic inflammation to regulate and inhibit T-cell functions. Cytotoxic T lymphocyte-associated antigen 4 (CTLA4), also called CD152, is a leucocyte differentiation antigen. Ligands of both CTLA4 and CD28 are B7 molecules, and CTLA4 after binding to a B7 molecule induces anergy of T-cells and is involved in negative regulation of immune responses. Abatacept (the trade name Orencia™, Bristol-Myers Squibb Company) which was marketed in 2005 and is useful for treating rheumatoid arthritis, is a monofunctional fusion protein which is formed by fusing two extracellular domains (ECD) of a CTLA4 molecule to a Fc fragment of human IgG1 and competitively blocks the binding of B7 to a CD28 molecule so as to inhibit proliferation and activation of T-cells (Genovese MC, Becker JC, SchiffM, et al. N. Engl. J. Med., 2005, 353: 1114-1123).

Interleukin 17A (IL-17 or IL-17A), a pro-inflammatory cytokine, is mainly produced from Th17 cells and is a most representative member of IL-17 family (Miossec P, Kolls JK. Nat. Rev. Drug. Discov., 2012, 11:763-776). IL-17A after binding to its receptor (IL-17RA), may induce various cells (such as fibroblasts, epithelial cells and endothelial cells) to express inflammatory cytokines and chemokines, and plays an important role in immune defense of a body (Lin Y, Ritchea S, Logar A. Immunity, 2009,31: 799-810). However, overexpressed IL-17A may cause inflammatory diseases. Synovial tissues of a patient with rheumatoid arthritis excessively express IL-17A, leading to the production of an important inflammatory factor - interleukin 6, which may be inhibited by an IL-17A neutralizing antibody (Chabaud M, Durand JM, Buchs N, et al. Arthritis Rheum. 1999, 42: 963-70). It has been demonstrated in many animal models with autoimmune diseases that pathological development of an inflammation may be effectively inhibited by neutralizing 1L-17A with an antibody (Lubberts E, Koenders MI, Oppers-Walgreen B, et al. Arthritis Rheum., 2004, 50: 650-659). At present, no antibody medicament associated with IL-17A has been approved to be marketed, but three medicaments are in a clinical research stage, namely Secukinumab (IL-17A-targeting antibody), Ixekizumab (IL-17A-targeting antibody) and Brodalumab (IL-17RA-targeting antibody). However, as shown by the results of clinical research of said medicaments, as to some chronic inflammatory diseases such as rheumatoid arthritis, these medicaments did not show an expected therapeutic effect (Kellner H, Ther Adv Musculoskelet Dis., 2013, 5: 141-152). Therefore, it might increase clinical benefits for patients with some chronic inflammations such as rheumatoid arthritis by using a treatment regime of combined administration. WO 2013/169338 A1 (MEDIMMUNE LLC [US]; MEDIMMUNE LTD [GB]) 14 November 2013) discloses a tetravalent CTLA-4 molecule that can be used against crohns disease, multiple rheumatoid arthritis,systemic lupus erythematosus or transplant rejection.

Combined administration requires sequential injections of two or more antibodies, or preparation of antibodies into a same dosage form. However, in an aspect, sequential injections of antibodies will reduce treatment compliance of a patient and increase pains. In another aspect, since different antibodies have different physicochemical properties, it is very difficult or almost impossible to prepare different antibodies into a same dosage form.

In view of this, it is still necessary to develop a new-type therapeutic medicament which simultaneously blocks B7/CD28 and IL-17A/IL-17RA signal pathways.

### SUMMARY OF THE INVENTION

The present invention provides a bifunctional fusion protein which simultaneously blocks B7/CD28 and IL-17A/IL-17RA signal pathways, a gene encoding the bifunctional fusion protein, a vector comprising the gene, a host cell comprising the vector, and a pharmaceutical composition containing the bifunctional fusion protein.

The first aspect of the present invention relates to a bifunctional fusion protein, comprising the extracellular region (i.e. extracellular domain) of a CTLA4 molecule and a functional fragment which neutralizes the activity of IL-17. In one embodiment, IL-17 is IL-17A.

In one embodiment, the bifunctional fusion protein further comprises a linker, wherein the extracellular domain of a CTLA4 molecule is linked to N-terminal or C-terminal of the functional fragment which neutralizes the activity of IL-17 via a linker. In one embodiment, the linker is 1-25 amino acids in length, *e.g.* 20, 21, 22, 23, 24 or 25 amino acids in length, *e.g.* the linker is an amino acid sequence as set forth in SEQ ID NO.3.

In one embodiment, the functional fragment which neutralizes the activity of IL-17 is an anti-IL-17 antibody or a functional fragment thereof, a chimeric antibody, a humanized antibody, a full humanized antibody, a single-chain antibody or a bispecific antibody. In one embodiment, the extracellular domain of a CTLA4 molecule is linked to N-terminal of a heavy chain or a light chain of an anti-IL-17 antibody via a linker. In some embodiments, the anti-IL-17 antibody is an IgG, IgA, IgD, IgE or IgM antibody, or a hybrid thereof. In some embodiments, the anti-IL-17 antibody is an IgG antibody.

The extracellular domain of the CTLA4 molecule comprises an amino acid sequence as set forth in SEQ ID NO.2, or an amino acid sequence having at least 70% identity thereto and having the same function, such as at least about 75%, 80%, 85%, 90%, 91%, 92%, 93%, 94%, 95%, 96%, 97%, 98%, 99%, 99.1%, 99.2%, 99.3%, 99.4%, 99.5%, or 99.6% identity.

In one embodiment, the heavy chain sequence of the anti-IL-17 antibody comprises an amino acid sequence as set forth in SEQ ID NO.28, or an amino acid sequence having at least 70% identity thereto and having the same function, such as at least about 75%, 80%, 85%, 90%, 91%, 92%, 93%, 94%, 95%, 96%, 97%, 98%, 99%, 99.1%, 99.2%, 99.3%, 99.4%, 99.5% or 99.6% identity. The light chain sequence of the anti-IL-17 antibody comprises an amino acid sequence as set forth in SEQ ID NO.27, or an amino acid sequence having at least 70% identity thereto and having the same function, such as at least about 75%, 80%, 85%, 90%, 91%, 92%, 93%, 94%, 95%, 96%, 97%, 98%, 99%, 99.1%, 99.2%, 99.3%, 99.4%, 99.5% or 99.6% identity.

In one embodiment, the extracellular domain of the CTLA4 molecule as set forth in SEQ ID NO.2 is linked to N-terminal of the heavy chain sequence of an anti-IL-17 antibody as set forth in SEQ ID NO.4 or 12 or to N-terminal of the light chain sequence of an anti-IL-17 antibody as set forth in SEQ ID NO.5 or 13 via a linker as set forth in SEQ ID NO.3.

In one embodiment, the extracellular domain of the CTLA4 molecule as set forth in SEQ ID NO.2 is linked to C-terminal of the heavy chain sequence of an anti-IL-17 antibody as set forth in SEQ ID NO.4 or 12 or to C-terminal of the light chain sequence of an anti-IL-17 antibody as set forth in SEQ ID NO.5 or 13 via a linker as set forth in SEQ ID NO.3.

In one embodiment, the amino acid sequence of the bifunctional fusion protein is selected from the group consisting of the following combinations: SEQ ID NOs.5 and 6, SEQ ID NOs.13 and 14, SEQ ID NOs.4 and 19, SEQ ID NOs.24 and 25, SEQ ID NOs.26 and 27, or an amino acid sequence having at least 70% identity thereto and having the same function, such as at least about 75%, 80%, 85%, 90%, 91%, 92%, 93%, 94%, 95%, 96%, 97%, 98%, 99%, 99.1%, 99.2%, 99.3%, 99.4%, 99.5% or 99.6% identity.

The second aspect of the present invention relates to a gene encoding the bifunctional fusion protein as described above.

In one embodiment, the encoding gene comprises nucleotide sequences as set forth in SEQ ID NOs.7 and 8, or SEQ ID NOs.15 and 16.

In some embodiments, nucleic acid sequences encoding a light chain and a heavy chain in the vector are present in different expression cassettes.

The third aspect of the present invention relates to a recombinant vector, comprising the above encoding gene operably linked therein.

In some embodiments, the vector is an eukaryotic cell expression vector. In some embodiments, the vector is the vector XOGC which has been modified to harbor two expression cassettes.

The fourth aspect of the present invention relates to a host cell, comprising the recombinant vector as described above.

The fifth aspect of the present invention relates to a method for preparing the bifunctional fusion protein as described above, comprising the following steps:
(1) operably linking the encoding gene as described above into an eukaryotic expression vector, and transfecting the same to a host cell for expression, or transfecting the recombinant vector as described above to a host cell for expression; and
(2) purifying the bifunctional fusion protein.

In one embodiment, the eukaryotic expression vector is XOGC. In one embodiment, the host cell is a HEK293-T cell or a CHO cell.

The sixth aspect of the present invention relates to a method for producing the bifunctional fusion protein as described above, comprising culturing the host cell as described above under appropriate conditions allowing for expression, and isolating and purifying the expressed protein.

The seventh aspect of the present invention relates to a pharmaceutical composition, comprising the bifunctional fusion protein as described above or a bifunctional fusion protein produced according to the method as described above.

In one embodiment, the bifunctional fusion protein as described above or the pharmaceutical composition as described above is useful for the prevention, treatment or amelioration of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

In another embodiment, the bifunctional fusion protein as described above or the pharmaceutical composition as described above is useful for the prevention, treatment or amelioration of rheumatoid arthritis, psoriasis, psoriatic arthritis, ankylosing spondylitis and Crohn's disease.

The eighth aspect of the present invention relates to use of the bifunctional fusion protein as described above, a bifunctional fusion protein produced according to the method as described above or the pharmaceutical composition as described above in the manufacture of a medicament for the prevention, treatment or amelioration of a disease.

In one embodiment, the disease is selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

The ninth aspect of the present invention relates to the use of bifunctional fusion protein for preventing, treating or ameliorating a disease, comprising a step of administering a therapeutically effective amount of the bifunctional fusion protein as described above, a bifunctional fusion protein produced according to the method as described above or the pharmaceutical composition as described above to a subject in need thereof.

In one embodiment, the disease is selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

The tenth aspect of the present invention relates to a kit which comprises the bifunctional fusion protein as described above, the nucleic acid molecule as described above, the vector as described above or the host cell as described above.

The present invention has the following advantages:
The experimental results of the present invention show that the bifunctional fusion protein of the present invention has a high expression level, strongly inhibits the secretion of inflammatory cytokines in *in vitro* experiments, and can effectively inhibit the development of arthritis in animal models. As known to one skilled in the art, the combination medication or fusion medication of some medicaments with different mechanisms may cause different effects, such as antagonism, irrelevance, accumulation or synergism, etc. Which interaction is presented by the combination medication or fusion medication of two or more medicaments can only be determined through specific experimental studies with a lot of work paid. Although it is likely to use the CTLA4 molecule and the anti-IL-17 antibody in combination to treat rheumatoid arthritis in the prior art, since a human body is complex, in particular, as to a systemic and immune disease such as rheumatoid arthritis of which pathogenesis is extremely complex and has not been understood thoroughly yet, even if medicaments are used in combination or in a fusion form, a fully accumulated or synergetic effect of the therapeutic effect achieved by each of medicaments with different mechanisms can also rarely be achieved. An irrelevant or even antagonistic effect is presented in many cases. An increased therapeutic effect on rheumatoid arthritis achieved by the bifunctional fusion protein of the present invention provides a candidate medicament which may have a more excellent therapeutic effect for the treatment for patients with rheumatoid arthritis, in particular patients with moderate and severe rheumatoid arthritis. Furthermore, the bifunctional fusion protein of the present invention simultaneously acts on two different targets for treating rheumatoid arthritis, reduces probability of treatment failure or a poor therapeutic effect of a therapy targeting a single target, and has an important economic significance and social benefits. Compared with the use of a monofunctional protein (e.g. CTLA4-Fc), the present invention could reduce production cost, decrease the volume and frequency of clinical administration and improve compliance of a subject, and has a promising application prospect in the prevention and treatment of immune diseases.

### BRIEF DESCRIPTION OF THE DRAWINGS

Figures 1: A and B show purification of the bifunctional fusion proteins A and B in the embodiments of the present invention with Protein A affinity chromatography, respectively.
Figures 2: A and B show purification of the bifunctional fusion proteins A and B in the embodiments of the present invention with ion exchange chromatography, respectively.
Figures 3: A and B show the monomer purities of the bifunctional fusion proteins A and B in the embodiments of the present invention, analyzed with size exclusion high-performance liquid chromatography, respectively.
Figure 4 shows binding affinities of the bifunctional fusion proteins A and C to human IL-17A.
Figure 5 shows binding affinities of the bifunctional fusion proteins A and C to mice IL-17A.
Figure 6 shows that the bifunctional fusion protein A inhibits IL-17A-induced secretion of GRO α from a HT-29 cell.
Figure 7 shows the immunosuppressive activity of the bifunctional fusion protein B measured with a mixed lymphocyte reaction.
Figure 8 shows the effect of a humanized analogue of the bifunctional fusion protein A on the reduction of rat CIA arthritis scores.

### DETAILED DESCRIPTION OF THE INVENTION

One objective of the present invention is to provide a bifunctional fusion protein comprising the extracellular domain of a CTLA4 molecule and a functional fragment which neutralizes the activity of IL-17. The extracellular domain of a CTLA4 molecule and the functional fragment which neutralizes the activity of IL-17 are operably linked, maintaining their respective spatial structures and exerting their respective physiological activity. The extracellular domain of a CTLA4 molecule and the functional fragment which neutralizes the activity of IL-17 may be directly fused to each other without affecting respective functions thereof. Furthermore, the extracellular domain of a CTLA4 molecule is linked to N-terminal or C-terminal of the functional fragment which neutralizes the activity of IL-17. Alternatively, other spacer sequences may be added therebetween, e.g. a linker.

As known to one skilled in the art, although the terms "comprise", "comprises", "comprising" or "comprised" is used when defining the bifunctional fusion protein, it does not mean that any other sequences irrelevant therewith in function may be arbitrarily added to the bifunctional fusion protein. When preparing a fusion protein having a complex composition such as a bifunctional fusion protein, in order to maintain the spatial structure, bioactivity and cell expression level of each component of the fusion protein, and in order to fuse each component together or in order to enhance the hydrolysis-resistant ability of the fusion protein, when preparing the fusion protein, one skilled in the art would add one or more additional amino acid residues among each component or to both terminals of the fusion proteins if required. Therefore, if the bifunctional fusion protein is defined in a closed expression way, these situations would not be truly covered.

The term "extracellular domain of a CTLA4 molecule" as used herein refers to an extracellular domain of cytotoxic T lymphocyte-associated antigen 4 (CTLA4). In a certain embodiment, an extracellular domain (ECD) sequence of a human CTLA4 protein is set forth in SEQ ID NO.2, an amino acid sequence from position 37 to position 161 of the human CTLA4 protein (Genbank accession number NM_005214.4).

In some embodiments, the term "functional fragment which neutralizes the activity of IL-17" as used herein refers to an immune globulin against IL-17, or a modifier, a functional equivalent, a functional fragment or a variant thereof. In some embodiments, the functional fragment which neutralizes the activity of IL-17 is an IgG antibody raised against IL-17. In some embodiments, the IgG is a chimeric, humanized or full human IgG. In some embodiments, the modifier may be a chemical modifier such as an acylated, alkylated or PEGylated product, as long as these modifiers retain the ability to target IL-17. In some embodiments, the functional equivalent refers to other polypeptide fragments capable of achieving the target binding ability of the immune globulin to IL-17. In some embodiments, the functional fragment refers to a protein fragment which retains the ability to target IL-17, such as a single-domain antibody, a single-chain antibody, a single chain variable fragment (scFv), a Fab fragment or F(ab')2 fragment. In some embodiments, the variant refers to a polypeptide derived from a parent protein through one or more changes, i.e. substitutions, insertions and/or deletions at one or more (some) positions.

In some embodiments, the linker is 5-30 amino acids in length. In some embodiments, the linker is 25 amino acids in length. In some embodiments, the sequence of the linker is GGGGSGGGGSGGGGSGGGGSGGGGS. The linker as used herein does not have special restrictions, as long as it can play a role in spacing two components of the fusion protein so as to make each component correctly form its respective spatial structure, and retain the bioactivity and the cell expression level thereof.

In some embodiments, the amino acid sequence of the bifunctional fusion protein is selected from the group consisting of the following combinations: SEQ ID NOs.5 and 6, SEQ ID NOs.13 and 14, SEQ ID NOs.4 and 19, SEQ ID NOs.24 and 25, SEQ ID NOs.26 and 27, or an amino acid sequence having the same function obtained from the sequence as described above through substitution, deletion or addition of one or more amino acid residues, such as 2, 3, 4, 5, 10, 15, 20, 30 and 50 amino acid residues, or an amino acid sequence having at least 70% identity thereto and having the same function, such as at least about 75%, 80%, 85%, 90%, 91%, 92%, 93%, 94%, 95%, 96%, 97%, 98%, 99%, 99.1%, 99.2%, 99.3%, 99.4%, 99.5% or 99.6% identity. A conservative amino acid of an amino acid is well known in the art.

The term "identity" as used herein has a meaning as generally known in the art. One skilled in the art also well knows the rules and standards for determining identities between different sequences. The sequences of the present invention defined with different identity degrees must simultaneously have the activity of the extracellular domain of a CTLA4 molecule or the activity to neutralize IL-17. One skilled in the art well knows the processes and means for screening a variant sequence with the above activities. One skilled in the art would readily obtain such a variant sequence under the teaching of the disclosure of the present invention.

In one embodiment, the present invention relates to an encoding gene comprising a nucleotide sequence encoding the bifunctional fusion protein as described above.

As known to one skilled in the art, although the terms "comprise", "comprises", "comprising" or "comprised" are used when defining the encoding gene, it does not mean that any other sequences irrelevant therewith in function may be arbitrarily added to both terminals of the encoding gene. One skilled in the art knows that in order to meet the recombination operation requirements, it is necessary to add appropriate restriction endonuclease cleavage sites to both terminals of the encoding gene, or additionally add start codon, stop codons and the like. Therefore, if the encoding gene is defined in a closed expression way, these situations would not be truly covered.

As known to one skilled in the art, in the case that the encoded amino acid is not changed, one or more codons in the encoding gene sequence may be equivalently substituted, e.g. one or some codons such as 1, 2, 3, 4, 5, 6, 8, 9, 10, 15, 20, 30 , 40 and 50 codons. Codon usage table is well known in the art.

In one embodiment, the present invention relates to a recombinant vector, comprising the encoding gene as described above which is operably linked therein. The recombinant vector may be a recombinant expression vector, prokaryotic or eukaryotic, but preferably a eukaryotic expression vector, more preferably a recombinant expression vector for mammalian eukaryotic expression.

The term "operably linked" used herein refers to such a linking mode, wherein the encoding gene is placed at an appropriate position to render the encoding gene correctly and successfully duplicated, transcribed or expressed.

In one embodiment, the present invention relates to a host cell into which the recombinant vector as described as above is transformed or transfected. The host cell includes a mammalian cell, a bacterial cell, a yeast cell, an insect cell and a plant cell.

In a preferred embodiment, the host cell includes a CHO cell, a HEK293 cell, a NSO cell and a SP2/0 cell.

In one embodiment, the present invention relates to a method for producing the bifunctional fusion protein, comprising the steps of culturing the above host cell under appropriate conditions for expression of a polypeptide, and isolating and purifying the expressed polypeptide from the cell medium. Preferably, the purified polypeptide has a purity of greater than 50%, more preferably greater than 60%, 65%, 70%, 75%, 80%, 85%, 90%, 95%, 96%, 97%, 98%, 99%, 99.5%, 99.6%, 99.7%, 99.8% or 99.9%.

In one embodiment, the present invention relates to a pharmaceutical composition, comprising the bifunctional fusion protein as described above and a pharmaceutically acceptable carrier. The bifunctional fusion protein may be the sole active ingredient, or may also be one of active ingredients of the pharmaceutical composition. Other active ingredients may be other therapeutic agents which can be used in combination with the bifunctional fusion protein.

In a preferred embodiment, the pharmaceutical composition of the present invention may be a single-dose dosage form, a topical dosage form or a systemic dosage form.

In one embodiment, the present invention relates to use of the bifunctional fusion protein as described above or the pharmaceutical composition as described above in the prevention, treatment or amelioration of a disease selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation. In some embodiments, the bifunctional fusion protein as described above or the pharmaceutical composition as described above is useful for the prevention, treatment or amelioration of a disease selected from a group consisting of rheumatoid arthritis, psoriasis, psoriatic arthritis, ankylosing spondylitis or Crohn's disease.

In some embodiments, the present invention relates to a method for preventing, treating or ameliorating a disease, comprising a step of administering a therapeutically effective amount of the bifunctional fusion protein as described above, the bifunctional fusion protein produced according to the method as described above or the pharmaceutical composition as described above to a subject in need thereof.

In one embodiment, the disease is selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

The term "therapeutically effective amount" refers to an amount that may exert a pharmacological effect in the body of a subject when administered thereto. "A therapeutically effective amount" could be readily determined by one skilled in the art according to the patient's conditions such as age, body weight and disease state.

In one embodiment, the present invention relates to use of the bifunctional fusion protein as described above, the bifunctional fusion protein produced according to the method as described above or the pharmaceutical composition as described above in the manufacture of a medicament for the prevention, treatment or amelioration of a disease.

In one embodiment, the disease is selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

As known to one skilled in the art, although the conditions which can be prevented, treated or ameliorated by the bifunctional fusion protein of the present invention have been listed above, the conditions which can be treated by the bifunctional fusion protein of the present invention are not limited to the above listed specific conditions. Any conditions which may be prevented, treated or ameliorated by simultaneously blocking B7/CD28 and IL-17A/IL-17RA signal pathways are falling within the scope of the present invention.

According to the above technical solutions of the present invention, the present invention has the following beneficial effects:
1) Blocking molecules of two signal pathways B7 and IL-17A (the extracellular domain of human CTLA4 and an anti-IL-17 antibody) are fused into a single molecule for expression and production. Compared with the production of extracellular domain of a CTLA4 protein and the anti-IL-17 antibody respective, it vastly reduces the operation and production cost.
2) When the extracellular domain of a human CTLA4 protein is linked to the anti-IL-17 antibody, the obtained protein of interest forms a tetramer, which not only meets the requirements for forming a dimer form to make the extracellular domain of CTLA4 exert its activity, but also keeps the titer of the anti-IL-17 antibody still divalent. Therefore, the total titer of the protein of interest is trivalence. Furthermore, as shown by the screening and comparison of the linking modes and *in vitro* binding assays, the operable linking mode in the present invention has no influence on the binding of the extracellular domain of the CTLA4 molecule to its ligand B7 and the binding of the anti-IL-17 antibody to IL-17.
3) After further modification, the bifunctional protein of the present invention may form a chimera or a humanized analogue, and further decrease its immunogenicity while maintaining the binding activity thereof.
4) The results of disease model experiments show that the bifunctional fusion protein can effectively relieve an inflammatory response of the disease animal model.

### Examples

### Example 1: Construction of the expression vector of the bifunctional fusion protein A

### (1) The amino acid sequences and encoding nucleotide sequences of the bifunctional fusion protein A

The signal peptide sequence used to express the bifunctional fusion protein A is set forth in SEQ ID NO.1; the extracellular domain sequence (ECD) of the human CTLA4 protein is set forth in SEQ ID NO.2, which is an amino acid sequence from position 37 to position 161 of the human CTLA4 protein (Genbank accession number NM_005214.4); the linker is (G₄S)₅ which has a sequence as set forth in SEQ ID NO.3; the variable region sequence of the anti-IL-17A antibody (control antibody A) in the bifunctional fusion protein A is from US patent NO. 7,846,443 (rat 1D10), and the constant region sequence is a constant region sequence of mice IgG2a, i.e. the heavy chain (hc) sequence of the anti-IL-17A antibody is set forth in SEQ ID NO.4 and the light chain (1c) sequence is set forth in SEQ ID NO.5; the amino acid sequence of the human CTLA4 ECD-linker-anti-IL-17AmAb hc-A is set forth in SEQ ID NO.6.

The nucleotide sequence which encodes the amino acid sequence as set forth in SEQ ID NO.6 is optimized according to the codon bias of mammalian cells, and the sequence obtained is set forth in SEQ ID NO.7. The nucleotide sequence which encodes the amino acid sequence as set forth in SEQ ID NO.5 is optimized according to codon bias of mammalian cells, and the sequence obtained is set forth in SEQ ID NO.8. The sequences as set forth in SEQ ID NO.7 and SEQ ID NO.8 have been synthetized by GenScript (Nanjing) Co., Ltd., which is introduced into the pUC57 vector by utilizing TA-cloning, and the resulting positive clones have been sequenced, verified, and named as pUC57-CTLA4-anti-IL-17A mAb hc-A and pUC57-anti-IL-17AmAb 1c-A, respectively.

### (2) Construction of the expression vector of the bifunctional fusion protein A and preparation of plasmids for transfection

The pUC57-CTLA4-anti-IL-17A mAb hc-A plasmid (GenScript (Nanjing) Co., Ltd.) is used as a template. The sequence which encodes the signal peptide-human CTLA4 ECD-linker-anti-IL-17AmAb hc-A is amplified through the conventional PCR. The upstream primer used comprises a *Hind* IIII enzyme cutting site, and the sequence is CCCAAGCTTGCCACCATGGGGGTCCTG (SEQ ID NO. 9). The downstream primer comprises a *EcoR* I enzyme cutting site, and the sequence is CCGGAATTCTCATTTGCCTGGGGTTCT (SEQ ID NO. 10).

The pUC57- anti-IL-17A mAb 1c-A plasmid (GenScript (Nanjing) Co., Ltd.) is used as a template. The sequence which encodes anti-IL-17A mAb lc-A is amplified through the conventional PCR. The upstream primer used comprises a *Hind* III enzyme cutting site, and the sequence is SEQ ID NO. 9. The downstream primer comprises an *Eco*R I enzyme cutting site, and the sequence is CCGGAATTCTCAGCACTCATTCCG (SEQ ID NO. 11).

The sequences encoding the signal peptide-human CTLA4 ECD-linker-anti-IL-17AmAb hc-A and the anti-IL-17A mAb lc-A obtained by amplification (1881 bp and 717 bp in length, respectively) are isolated through 1% agarose gel electrophoresis and then the corresponding fragments are recovered. The gene fragments recovered are introduced into the eukaryotic expression vector XOGC (US publication NO.20100120089) after being subjected to *Hind* III and *EcoR* I digestion, to obtain recombinant plasmids X0GC-CTLA4-anti-IL-17AmAb hc-A and X0GC-anti-IL-17A mAb lc-A, which are then respectively transformed into *Escherichia coli* DH5 α to obtain recombinant bacteria strains DH5 α /X0GC-CTLA4-anti-IL-17AmAb hc-A and DH5 α /X0GC-anti-IL-17AmAb lc-A. Positive clones are screened through PCR and DNA sequencing is conducted so as to verify that the recombinant plasmids are correctly constructed.

The positive strains DH5 α /X0GC-CTLA4-anti-IL-17AmAb hc-A and DH5 α /X0GC-anti-IL-17AmAb lc-A are respectively seeded into 1L LB/Amp liquid medium (composed of 1% peptone (BD company), 0.5% yeast extract (BD company) and 1% NaCl (Sinopharm Chemical Reagent Co., Ltd.)), and incubated overnight with 180 rpm shaking speed at 37°C. On the second day, plasmids are extracted by using an EndoFree Plasmid Maxi Kit (Qiagen, 12381) for transfecting HEK293-T cells (Cell Resource Center of Shanghai Institutes for Biological Sciences, Chinese Academy of Sciences).

### Example 2: Construction of the expression vector of the bifunctional fusion protein B

### (1) The amino acid sequences and encoding nucleotide sequences of the bifunctional fusion protein B

The signal peptide sequence used to express the bifunctional fusion protein B is set forth in SEQ ID NO.1; the ECD sequence of the human CTLA4 protein as set forth in SEQ ID NO.2 is an amino acid sequence from position 37 to position 161 of the human CTLA4 protein (Genbank accession number NM_005214.4); the linker is (G₄S)₅ which has a sequence as set forth in SEQ ID NO.3; the sequence of the anti-IL-17A antibody in the bifunctional fusion protein B is from US patent No. 7,846,443 (humanized 16C10), of which the heavy chain (hc) sequence is set forth in SEQ ID NO.12 and the light chain (1c) sequence is set forth in SEQ ID NO.13; the amino acid sequence of the human CTLA4 ECD-linker-anti-IL-17AmAb hc-B is set forth in SEQ ID NO. 14.

The nucleotide sequence which encodes the amino acid sequence as set forth in SEQ ID NO.14 is optimized according to the codon bias of mammalian cells, and the sequence obtained is set forth in SEQ ID NO.15. The nucleotide sequence which encodes the amino acid sequence as set forth in SEQ ID NO.13 is optimized according to the codon bias of mammalian cells, and the sequence obtained is set forth in SEQ ID NO.16. The sequences as set forth in SEQ ID NO.15 and SEQ ID NO.16 have been synthetized by GenScript (Nanjing) Co., Ltd., which is introduced into the pUC57 vector by utilizing TA-cloning, and the resulting positive clones have been sequenced, verified and named as pUC57-CTLA4-anti-IL-17A mAb hc-B and pUC57-anti-IL-17AmAb lc-B, respectively.

### (2) Construction of the expression vector of the bifunctional fusion protein B and preparation of plasmids for transfection

The pUC57-CTLA4-anti-IL-17A mAb hc-B plasmid (GenScript (Nanjing) Co., Ltd.) is used as a template. The sequence which encodes the signal peptide-human CTLA4 ECD-linker-anti-IL-17AmAb hc-B is amplified through the conventional PCR. The upstream primer used comprises a *Hind* III enzyme cutting site, and the sequence is SEQ ID NO. 9. The downstream primer comprises a *EcoR* I enzyme cutting site, and the sequence is CCGGAATTCTCACTTTCCTGGTGACAGACTCA (SEQ ID NO. 17).

The pUC57- anti-IL-17A mAb lc-B plasmid (GenScript (Nanjing) Co., Ltd.) is used as a template. The sequence which encodes anti-IL-17A mAb lc-B is amplified through the conventional PCR. The upstream primer used comprises a *Hind* III enzyme cutting site, and the sequence is SEQ ID NO. 9. The downstream primer comprises a *Eco*R I enzyme cutting site, and the sequence is CCGGAATTCTCAGCACTCGCCCCGGTTG (SEQ ID NO. 18).

The sequences encoding the signal peptide-human CTLA4 ECD-linker-anti-IL-17AmAb hc-B and the anti-IL-17A mAb lc-B obtained by amplification (1887 bp and 735 bp in length, respectively) are isolated through 1% agarose gel electrophoresis and then the corresponding fragments are recovered. The gene fragments recovered are introduced into the eukaryotic expression vector XOGC (US publication No. 20100120089) after being subjected to *Hind* III and *EcoR* I digestion, to obtain recombinant plasmids X0GC-CTLA4-anti-IL-17A mAb hc-B and X0GC-anti-IL-17A mAb lc-B, which are then respectively transformed into *Escherichia coli* DH5 α to obtain recombinant bacteria strains DH5 α /X0GC-CTLA4-anti-IL-17AmAb hc-B and DH5 α /X0GC-anti-IL-17AmAb lc-B. Positive clones are screened through PCR and DNA sequencing is conducted so as to verify that the recombinant plasmids are correctly constructed.

The positive strains DH5 α /X0GC-CTLA4-anti-IL-17AmAb hc-B and DH5 α /X0GC-anti-IL-17AmAb lc-B are respectively seeded into 1L LB/Amp liquid medium (composed of 1% peptone (BD company), 0.5% yeast extract (BD company) and 1% NaCl (Sinopharm Chemical Reagent Co., Ltd.)), and incubated overnight with 180rpm shaking speed at 37°C. On the second day, plasmids are extracted by using an EndoFree Plasmid Maxi Kit (Qiagen, 12381) for transfecting HEK293-T cells.

### Example 3: Expressions of the bifunctional fusion protein A and the bifunctional fusion protein B

HEK293-T cells in a good growth status with vitality (ratio of viable cells) above 95% are seeded in a 10-layer cell factory (NUNC Company, product number: 140400) in an amount of 1.8 x 10⁷ cells, and are cultured in a DMEM medium (Corning Company) supplemented with 10% fetal bovine serum (Gibco Company). The cell factory is mixed thoroughly by inverting and then is placed in a 37°C incubator at 5% CO₂, and grown for 48 hours. When cells completely adhere to the wall and the confluence reaches 80%, they can be used for a transient transfection.

The plasmids X0GC-CTLA4-anti-IL-17A mAb hc-A and X0GC-anti-IL-17A mAb 1c-A are simultaneously used to transfect cells to express the bifunctional fusion protein A. The plasmids X0GC-CTLA4-anti-IL-17A mAb hc-B and X0GC-anti-IL-17A mAb lc-B are simultaneously used to transfect cells to express the bifunctional fusion protein B. After filtrated with a 0.22 µ m filter membrane, 1330µg each transfection mixture is pipetted and then added to 66ml serum-free DMEM medium. 2660 µg transfection reagent PEI (Sigma Company) is pipetted and then added to 66ml serum-free DMEM medium. After mixed respectively, the resulting PEI mixture liquid is poured into the DMEM comprising plasmids and then mixed. After standing at room temperature for 15 minutes, the mixture comprising plasmids and PEI is added to 1.3L serum-free DMEM medium, mixed thoroughly and slowly added to the cell factory. The cell factory is placed in a 37°C incubator at 5% CO₂ and cultured. Four hours later, 266 ml Cell Boost 5 (Thermo Fisher Company) is added and mixed, and then further cultured for 6 days. Centrifuged at 7000 rpm for 20 min, the supernatant is pooled for the following protein purification. The concentrations of the supernatant are determined via ELISA. The protein expression level of each batch of the bifunctional fusion protein A and bifunctional fusion protein B is 4-8 mg/L.

### Example 4: Purification of the bifunctional fusion protein A and the bifunctional fusion protein B

The supernatant from the cell factory is filtered via a 0.2 µm filter membrane. Through ultrafiltration and concentration with a membrane cassette (nominal molecular weight cut off 10kDa), the solution is replaced with 20 mM a phosphate solution (pH 7.4) comprising 150 mM NaCl. Before subjected to a chromatographic column, the supernatant is filtered with a 0.2 µm filter membrane to remove the precipitates. This step is carried out at 4°C.

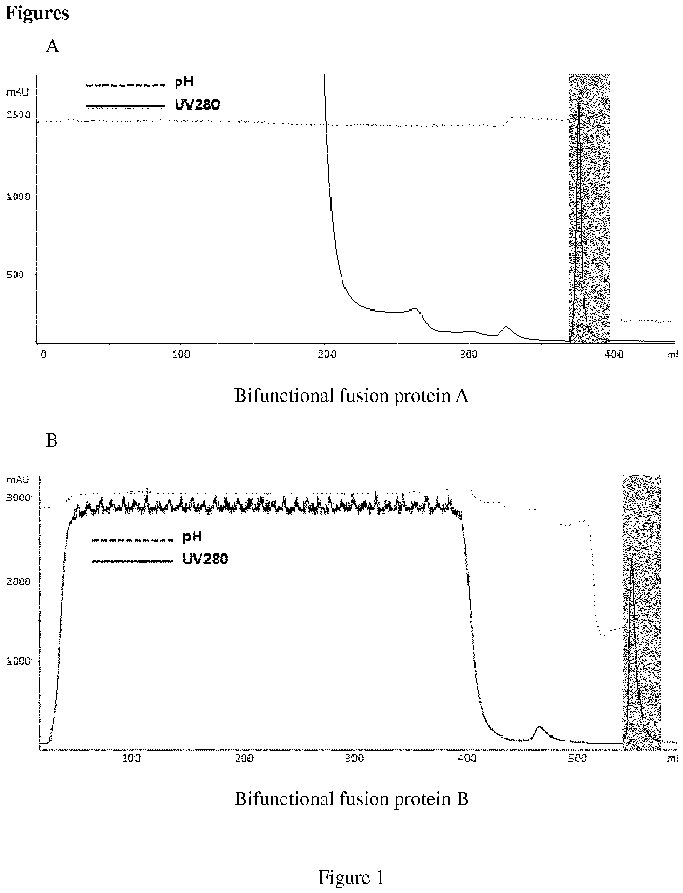

Purification step is carried out at 4°C by using AKTA explorer 100 type protein purification system (GE Healthcare) and affinity chromatographic column rProtein A Sepharose Fast Flow (16 mm I.D., 22 ml, GE Healthcare). First of all, the chromatographic column is equilibrated with mobile phase A (20 mM sodium phosphate buffer, 150 mM NaCl, pH 7.4). After the baseline becomes stable, the cell supernatant as treated above is loaded onto the column with a flow rate of 5 ml/min, followed by washing with mobile phase A. After that, the column is washed with 5-column volume of mobile phase B1 firstly (the mobile phase A comprising 0.5M arginine). Then, 5-column volume of mobile phase B2 (100 mM citric acid, pH 3.0) is used to elute the column, and elution peaks are collected as protein peaks of interest. The flow rate of the above eluting step is 5 ml/min. Chromatograms are shown in Figures 1A and B. The elution peaks indicated are collected (gray areas of Figures 1A and B), and pH is adjusted to 5.0 through the addition of 1 M sodium acetate solution.

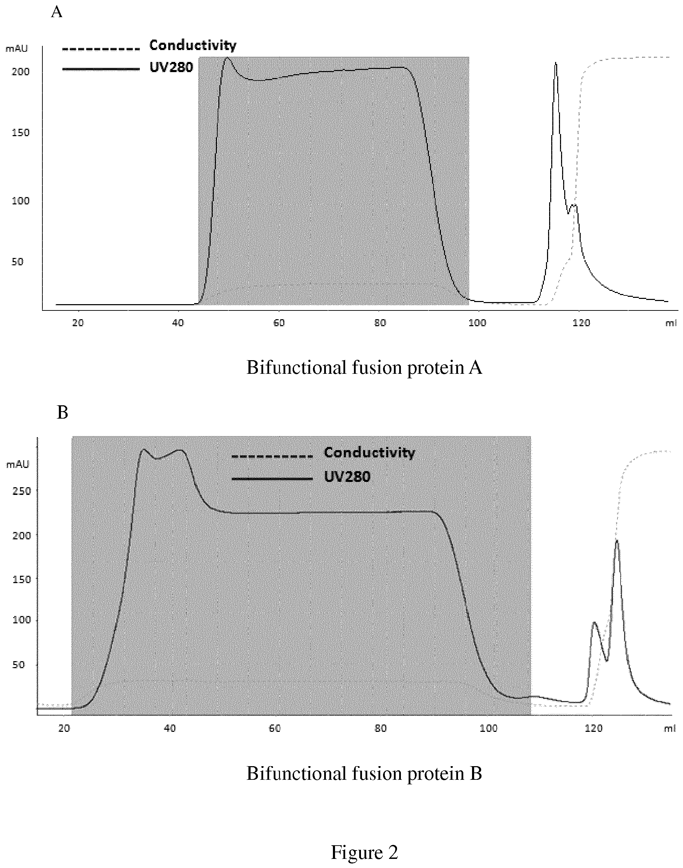

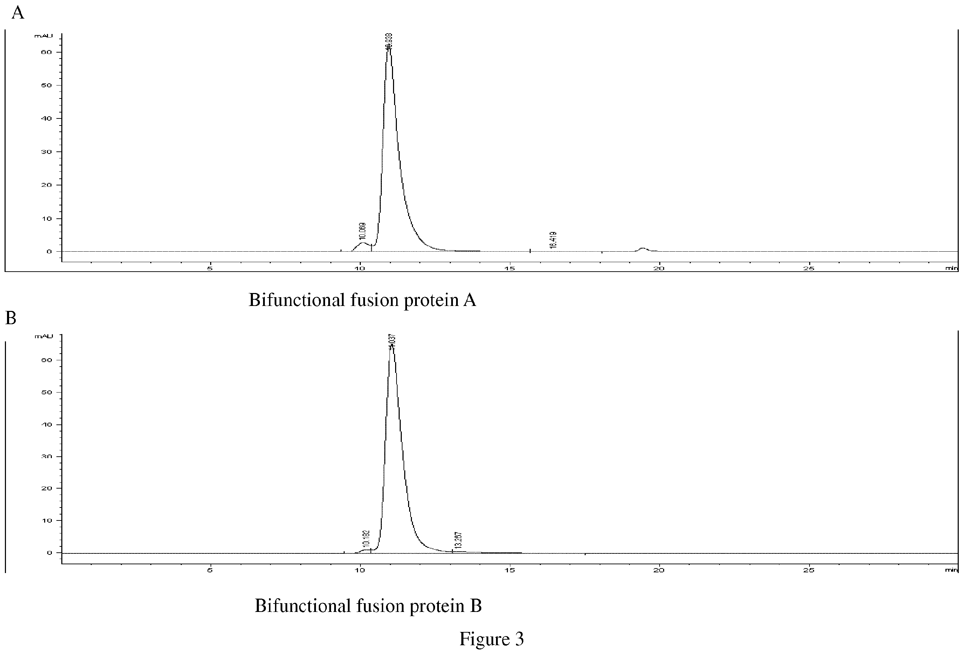

Purification step is carried out at 4°C by using AKTA explorer 100 type protein purification system (GE Healthcare) and strong anion exchange chromatographic column HiTrap Q Sepharose FF (5ml, GE Healthcare). First of all, the chromatographic column is equilibrated with mobile phase A (20 mM sodium acetate, pH 5.0). After the baseline becomes stable, the eluent collected and pH adjusted in the above experiment is loaded onto the column with a flow rate of 5 ml/min. Flow-through peaks are collected as protein peaks of interest. After the sample loading, isocratic elution is carried out with mobile phase B (20 mM sodium acetate, 1 M NaCl, pH 5.0) with a flow rate of 5 ml/min. Chromatograms are shown in Figures 2A and B. The fractions indicated are collected (gray areas of Figures 2A and B), followed by concentration with a ultrafiltration tube (nominal molecular weight cut off 30kDa) and aseptic filtration with a 0.2 µm filter membrane in a laminar flow bench. Then an ultraviolet spectrophotometer (280 nm) is used to determine the contents of the protein, followed by purity analysis with a size exclusion high performance liquid chromatography (SE-HPLC). The results are indicated in Figures 3A and B. The purities of the bifunctional fusion protein A and the bifunctional fusion protein B are 96.6% and 97.4%, respectively.

### Example 5: Construction of cell lines stably expressing the bifunctional fusion proteins

After thawing, CHO/DG44 (dhfr-) cells are cultured for 2-3 passages. When the survival rate reaches 95%, they are used for the transfection experiments. Transfection of CHO/DG44 cells is carried out with transfection reagent Lipofectamine 2000 (Invitrogen, 11668019) according to the instruction thereof. Specifically, 5 µg of the plasmid and 3 mL α -MEM medium (SAFC, 51451C) are mixed in a centrifuge tube while 20 µ L the transfection agent and 3 mL α -MEM medium are mixed in another centrifuge tube. After standing at the laminar flow bench at room temperature for 5 min, the above diluted plasmid and the transfection agent are mixed together and further stand for 30 min. Then 6 mL the mixture is slowly added to a 75 cm² Rectangular Canted Neck cell culture flask (comprising CHO/DG44 cells with a confluence of 70-80%) which is then placed in a cell incubator (37°C and 5% CO₂). After cell growth overnight, the transfection agent mixture is removed, and a fresh α -MEM medium is added followed by adding 10% dialyzed FBS (Gibco, 30067-334). Then, the flask is placed in a cell incubator (37°C and 5% CO₂) and incubated for 1-2 days. After that, 1 mg/mL G418 (Cellgro, 61-234 RG) is added to the fresh medium, which is used to culture the cells for 2 weeks.

In order to obtain a monoclonal cell strain, a limiting dilution analysis is required. Specifically, a α -MEM medium supplemented with 10% dialyzed FBS and 1 mg/mL G418 is used to dilute the cell suspension to 5 cells/mL which is then seeded to a 96 well plate. The cells are placed in a cell incubator (37°C and 5% CO₂) for growing till a single cell grows into a single colony. The colonies from single cells are trypsinized and passaged to a 24 well plate. Then the cells are successively passaged to a 12 well plate and a 6 well plate. When the confluence of the monoclonal cell strain in the 6 well plate reaches 90%, the expression level of the protein of interest is measured with ELISA, and clones with a higher expression level are obtained, namely clone No.52 (14 mg/L), clone No. 85 (56 mg/L) and clone No. 103 (59 mg/L).

### Example 6: Small-scale fermentation of the bifunctional fusion proteins

Clone No. 103 is selected for a fermentation in a 5L bioreactor (Sartorius, Bplus twin 5L). It is seeded by 5 x10⁵ cells to Hyclone SFM4CHO medium (4 g/L glucose and 0.3 g/L glutamine). The bioreactor has a working volume of 2.4L, and the starting process parameters are as follows: 37°C, pH 7.1, dissolved oxygen 40%-60% and the stirring speed 40 rpm. Afterwards, the stirring speed is increased by 5 rpm per day. Furthermore, after 5 days, the temperature is lowered with a speed of 0.5°C/h until 34 °C, and 0.3 mM sodium butyrate is added. Two weeks later, the fermentation broth is harvested and centrifuged at 7000 rpm for 20 min. The supernatant is harvested and kept. Upon ELISA assay, the concentration is determined to be 240 mg/L.

### Example 7: Binding affinities of the bifunctional fusion proteins A and C to human IL-17A and mouse IL-17A

In the binding affinity experiments, the bifunctional fusion protein C is constructed which is similar to the bifunctional fusion proteins A. The amino acid sequence of the bifunctional fusion protein C is set forth in SEQ ID NO. 19 (CTLA4 ECD-linker- anti-IL-17AmAb lc) and SEQ ID NO. 4, that is, CTLA4 ECD is linked to the light chain of anti-IL-17AmAb via a linker (i.e. linked to SEQ ID NO. 5), different from the bifunctional fusion protein A of which CTLA4 ECD is linked to the heavy chain of anti-IL-17AmAb via a linker (i.e. linked to SEQ ID NO. 4). In addition, control antibody A serves as a control antibody of the bifunctional fusion proteins A and C, of which the amino acid sequences are set forth in SEQ ID NO. 4 (the heavy chain of the control antibody A) and SEQ ID NO. 5 (the light chain of the control antibody A). That is, the control antibody A corresponds to the anti-IL-17A antibody moiety in the bifunctional fusion proteins A and C.

The method is specifically implemented as follows. Human IL-17A (Sino Biological Inc., product No. 12047-HNAE) or mouse IL-17A (Sino Biological Inc., product No. 51065-MANE) is coated onto a 96 well high-adsorption ELISA plate (Corning, 2592) with a sodium carbonate solution (15 mM sodium carbonate, 35 mM sodium bicarbonate, and pH 9.6) with a coating concentration 1 µ g/mL, 100 µ L/well, and coated overnight at 4°C. The plate is washed with PBST (Sigma, product No. P-3563) for 5 times. The plated is blocked with PBST comprising 1% BSA, 300 µ L/well, and incubated at 25 °C for at least µ hour. The plate is washed with PBST for 5 times. Samples to be tested is added at a specific concentration, which are diluted in PBST comprising 1% BSA, 100 µ L/well, and incubated at 25 °C for 1 hour. The plate is washed with PBST for 5 times. Then a horse radish peroxidase labeled anti-mouse IgG antibody (1:10,000, in PBST comprising 1% BSA) (Abcam, product No. Ab7068) is added, 100 µ L/well, and incubated at 25 °C for 1 hour. The plate is washed with PBST for 5 times. Colorimetric substrate TMB (BD OptEIA, product No.555214) is added, 100 µ L/well, and colorimetric reaction is developed at room temperature for 10 min. 1M H₂SO₄ is added with 100 µL/well to terminate the colorimetric development. The Absorbance at 450 nm is read on ELISA.

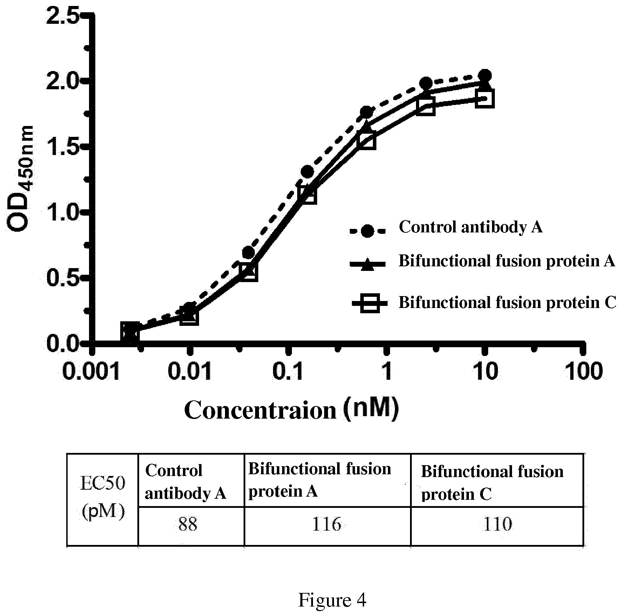

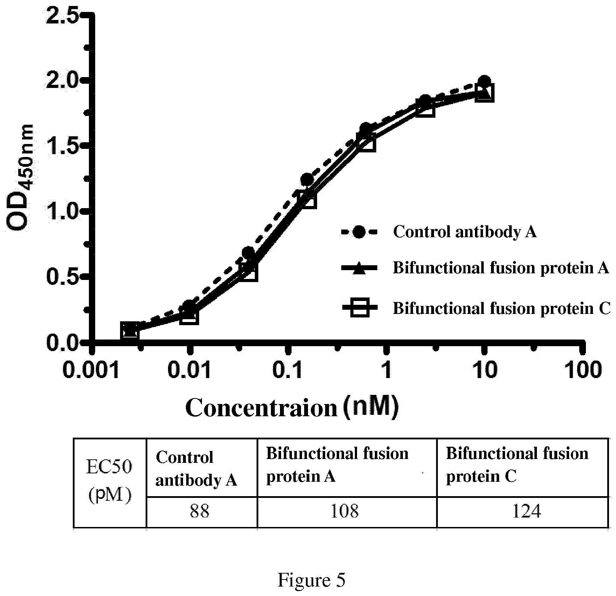

The results are shown in Figures 4 and 5. The bifunctional fusion proteins A and C in the present invention have high binding affinities to their antigens human IL-17A (Figure 4) and mouse IL-17A (Figure 5), which are similar to that of the control antibody A. The above results show that CTLA4 ECD linked to N-terminal of the heavy chain or light chain of the control antibody A via a linker does not significantly affect the affinity of the control antibody A to its antigen.

In addition, it shall be noted that the bifunctional fusion protein B in the present invention cannot bind to mouse IL-17A, but has a high binding affinity to human IL-17A (the value measured with ELISA assay in the present invention is EC50=103 pM), which is close to that in US patent No. 7846443.

### Example 8: Influences of different linking modes of CTLA4 on the affinity of a bifunctional fusion protein to B7

In order to study and compare the influences of different linking modes of CTLA4 on the affinity of a bifunctional fusion protein to B7, we construct analogues of the bifunctional fusion protein having different CTLA4 linking modes:
(1) The bifunctional fusion protein C: as described above, its sequences are SEQ ID NO. 19 (CTLA4 ECD-linker- anti-IL-17AmAb lc) and SEQ IDNO. 4, i.e., CTLA4 ECD is linked to N-terminal of the light chain of anti-IL-17AmAb via a linker (i.e. linked to SEQ ID NO. 5), different from the bifunctional fusion protein A of which CTLA4 ECD is linked to N-terminal of the heavy chain of anti-IL-17AmAb via a linker (i.e. linked to SEQ ID NO. 4).
(2) The bifunctional fusion protein D: the signal peptide sequence used is set forth in SEQ ID NO.1; the extracellular domain sequence (ECD) of the human CTLA4 protein is set forth in SEQ ID NO.2; the linker is (G₄S)₅ which has a sequence as set forth in SEQ ID NO.3; the heavy chain (hc) sequence of the anti-IL-17A antibody is set forth in SEQ ID NO.4 and the light chain (1c) sequence is set forth in SEQ ID NO.5; the amino acid sequence of anti-IL-17AmAb hc-A-linker-CTLA4 ECD is set forth in SEQ ID NO.20, i.e. the CTLA4 of the bifunctional fusion protein D is linked to C-terminal of the heavy chain of the control antibody A via a linker.
(3) The bifunctional fusion protein E: the signal peptide sequence used is set forth in SEQ ID NO.1; the extracellular domain sequence (ECD) of the human CTLA4 protein is set forth in SEQ ID NO.2; the linker is (G₄S)₅ which has a sequence as set forth in SEQ ID NO.3; the heavy chain (hc) sequence of the anti-IL-17A antibody is set forth in SEQ ID NO.4 and the light chain (lc) sequence is set forth in SEQ ID NO.5; the amino acid sequence of anti-IL-17AmAb lc-A-linker-CTLA4 ECD is set forth in SEQ ID NO.21, i.e. the CTLA4 of the bifunctional fusion protein E is linked to C-terminal of the light chain of the control antibody A via a linker.

CTLA4 ECD can bind to its ligand - B7 molecule. In the experiments for measuring the binding affinity to B7, the binding abilities of the bifunctional fusion proteins A, C, D and E to the B7 molecule are measured on Raji cells which highly express the B7 molecule with flow cytometry. This experiment is specifically carried out as follows. Centrifuging at 1000 rpm for 5 min to collect Raji cells (ATCC, product No. CCL-86), washing with PBS (PromoCell, product No. C-40232) comprising 2% FBS (GIBCO, product No. 10099141) once. Resuspending Raji cells by 1x10⁶ cells per tube in 200 µ L cold PBS which comprises 2% FBS and the samples to be tested at a final concentration of 0.5 nM in a tubes. Incubating the a tubes on ice for 30 min and then washing the cells with PBS comprising 2% FBS twice. Then resuspending the cells in 200 µ L cold PBS comprising 2% FBS and an FITC labeled anti-mouse IgG antibody (1:50 diluted). Incubating the tubes on ice in the dark for 30 min and then washing the cells with PBS comprising 2% FBS twice. Resuspending the cells in 500 µ L cold PBS comprising 2% FBS and subjecting the cell suspension onto a Flow Cytometer for detection and analysis.

The results are shown in Table 1. The bifunctional fusion protein A has a binding ability to Raji cells highly expressing its ligand - B7 molecule, which is significantly greater than those of the bifunctional fusion proteins C, D and E. The results above show that different linking modes of CTLA4 ECD to the anti-IL-7A antibody may significantly affect its binding ability to B7.

**Table 1. Binding abilities of the bifunctional fusion proteins to Raji cells highly expressing B7 molecule**

| **Samples** | **Mean fluorescence intensity (MFI)** |
|---|---|
| IgG negative control | 5 |
| Bifunctional fusion protein A | 51 |
| Bifunctional fusion protein C | 28 |
| Bifunctional fusion protein D | 9 |
| Bifunctional fusion protein E | 3 |

### Example 9: The bifunctional fusion protein A inhibits GROα secretion from HT-29 cells induced by IL-17A

Human colorectal cancer cell strain H-29 is from ATCC. HT-29 cells are cultured in RPMI-1640 medium (Gibco, product No. 22400) supplemented with 10% fetal bovine serum (Gibco, product No. 10099), in a 37°C cell incubator at 5% CO₂. The cells are passaged twice per week.

The specific procedures for inhibiting GROα secretion induced by IL-17A are as follows. Gradiently diluting the samples to be tested in RPMI 1640 complete medium supplemented with 10% fetal bovine serum, 50 µL per well of the cell culture plate (Coring, product No. 3599). Adding human IL-17A diluted in the same complete medium at a concentration of 240 ng/mL to the cell culture plate, 50 µL per well. Incubating the cell culture plate in a 37°C incubator at 5% CO₂. Then resuspending the HT-29 cells in the complete medium and seeding them by 100 µL per well into a 96 well cell culture plate, i.e. about 20,000 cells. Incubating the cells in the 37°C incubator at 5% CO₂ for 48 h. Centrifuging the cell culture plate at 1000 rpm for 5 min and collecting the culture supernatant. Determining GROα level with a GROα ELISA kit (R&D, product No. DY275) in accordance with the instruction.

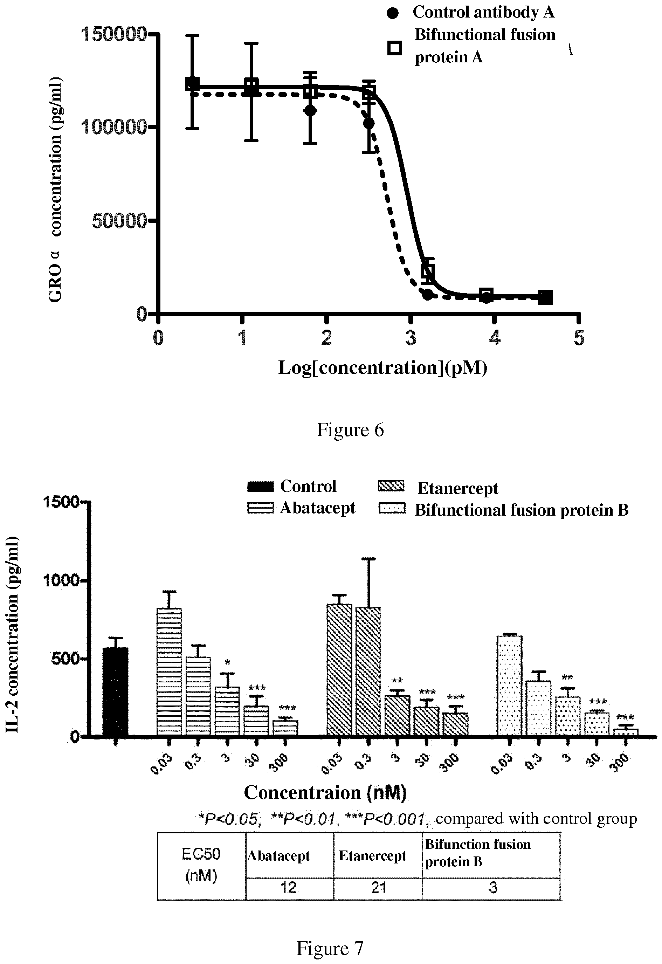

The results are shown in Figure 6. Both the control antibody A and the bifunctional fusion protein A can inhibit GROα secretion from HT-29 cells induced by IL-17A, of which IC₅₀ are 532 pM and 911 pM, respectively.

### Example 10: The bifunctional fusion protein B inhibits IL-2 secretion from human peripheral blood mononuclear cells

The immunosuppressive activity of the bifunctional fusion protein B of the present invention, i.e., an activity for inhibiting T lymphocyte activation, is measured with the mixed lymphocyte reaction assay. In brief, two human peripheral blood mononuclear cells (PBMC) samples are obtained from two different individuals, wherein one is inactivated to serve as stimulator cells and the other serves as responder cells. These two PBMC samples are mixed. The stimulator cells can activate immune responses of the responder cells, and secrete a large number of cytokine IL-2. The samples to be tested are co-incubated with this mixed PBMC system and the influence thereof on the IL-2 secretion is determined.

The specific protocol of the mixed lymphocyte reaction assay is as follows. Thawing two PBMC samples from different batches and from different individuals (Lonza, product No. CC-2702) and resuspending them in RPMI-1640 complete medium (Gibco, product No. 22400) supplemented with 10% fetal bovine serum (Gibco, product No.10099). One PBMC sample serves as stimulator cells, and the other PBMC sample serves as responder cells. Co-incubating the stimulator cells with 50 µg/ml mitomycin C (Wako, product No. 50-07-7) at 37°C for 45 min and then washing the cells with DPBS (PromoCell, product No. C-40232) twice and then resuspending the cells in RPMI 1640 complete medium. Counting the stimulator cells and the responder cells (Innovatis Cedex) and determining cell viability (Trypan blue dye, Invitrogen Company, product No. 15250-061). Then seeding the respondor cells by 50 µL per well (triplicated) to a 96 well cell culture plate (Corning, product No. 3799), i.e. 1x10⁵ cells/well, followed by adding 100 µL the bifunctional fusion protein B at a given concentration, commercialized positive control medicament Abatacept (Orencia™, Bristol-Myers Squibb Company) or Etanercept (Yi Sai Pu™, Shanghai CP Guojian Pharmaceutical Co., Ltd.). Adding 50 µL the stimulator cells to each well by 1x10⁵ cells per well. Incubating the cell culture plate in a 37°C incubator at 5% CO₂. After 72 hours, pipetting 100 µL culture supernatant from each well and stored at -70°C. Determining IL-2 level with an IL-2 ELISA kit (Raybiotech, product No. ELH-IL2-001) in accordance with the instruction.

The results are shown in Figure 7. In the mixed human PBMC reaction assay, EC50 of the bifunctional fusion protein B, Abatacept and Etanercept for inhibiting IL-2 secretion are 3 nM, 12 nM and 21 nM, respectively, showing that the bifunctional fusion protein B displays a stronger activity for inhibiting IL-2 secretion than those of the commercialized positive control medicaments Abatacept and Etanercept. Additional tests also show that the bifunctional fusion protein A also displays a stronger activity for inhibiting IL-2 secretion, which is close to the activity of Abatacept.

### Example 11: Chimeras and humanized analogues of the bifunctional fusion protein A

The amino acid sequence of the constant region of the bifunctional fusion protein A, i.e. the amino acid sequence of the constant region of the control antibody A is replaced with a sequence of the constant region of human IgG1 . The sequence of the heavy chain constant region of human IgG1 is set forth in SEQ ID NO. 22, and the sequence of the light chain constant region is set forth in SEQ ID NO. 23. A chimera analogue of the bifunctional fusion protein A is obtained therefrom, of which the amino acid sequences are set forth in SEQ ID NO. 24 and SEQ ID NO. 25. The analogue has the reduced mouse-derived ingredients (i.e. sequence) and thus the reduced immunogenicity of the protein.

In addition, partial sequences of SEQ ID NO. 24 and SEQ ID NO. 25 are further modified to replace and mutate the amino acids in the framework region in the variable region thereof so as to further reduce the mouse-derived ingredients. The amino acid sequences of this humanized analogues of the bifunctional fusion protein A obtained therefrom are set forth in SEQ ID NO. 26 and SEQ ID NO. 27.

The antigen binding affinities of the bifunctional fusion protein A, the chimera and the humanized analogues thereof are determined. The results are shown in Table 2, showing that both the chimera and the humanized analogues can effectively retain the antigen binding activity of the parent sequences.

**Table 2. Binding affinities of the bifunctional fusion protein A, the chimera and the humanized analogue to human IL-17A and B7**

| Protein | EC50 for binding to IL-17A | Mean fluorescence intensity (MFI) for binding to B7 |
|---|---|---|
| Bifunctional fusion protein A | 201 | 21 |
| Chimera Analogue | 382 | 20 |
| Humanized Analogue | 460 | 20 |

### Example 12: Pharmacodynamics studies of the humanized analogue of the bifunctional fusion protein A in the II type collagen induced rat arthritis (CIA) model

The rat CIA model is a model generally used in the pathogenesis studies of rheumatoid arthritis and screening of therapeutic medicaments. 7 week-old female Wistar rats (purchased from Beijing HFK Bioscience Co., Ltd., P.R. China) are used as the experimental animals. After acclimatization for one week, 5 rats are randomly selected as normal control rats, and the rest of rats are used to establish a rat CIA model. The CIA model is obtained through the primary immunization and booster immunization. As to the primary immunization, 200 µg bovine collagen type II (Chondrex, product No. 20022) and Freund's incomplete adjuvant (Sigma-Aldrich, product No.F5506) are mixed to form an emulsion which is then injected intradermally at the end of the tail of rats. After one week, booster immunization is carried out. With respect to the booster immunization, 100 µg bovine collagen type II and the Freund's incomplete adjuvant are mixed to form an emulsion which is then injected intradermally at the end of the tail of rats. After the booster immunization, when four limbs of the rats are observed to manifest clinical symptoms of arthritis such as redness and edema of the paws, they are randomly divided into groups with 6 rats in each group.

Each group of the CIA model rats are administered with following agents respectively: a solvent (PBS), Abatacept (272nmol/kg, administered on 1, 5, 8 and 11 day, respectively), a humanized analogue of the bifunctional fusion protein A (33nmol/kg, administered every other day intraperitoneally, 7 times in total). After administration, pathological changes of the four paws are observed, and the severity of arthritis is scored: 0 = no evidence of redness and edema; 1= erythema and mild edema confined to ankle joint or tarsal joint; 2= erythema and mild edema from the ankle joint to the tarsal joint; 3= erythema and moderate edema from the ankle joint to the metatarsal joint; and 4= erythema and severe edema, from the ankle to paws including phalangeal joint or ankylosis of four limbs. Inflammation scores are given to four limbs of the rats, and the maximum score for each rat is 16 scores.

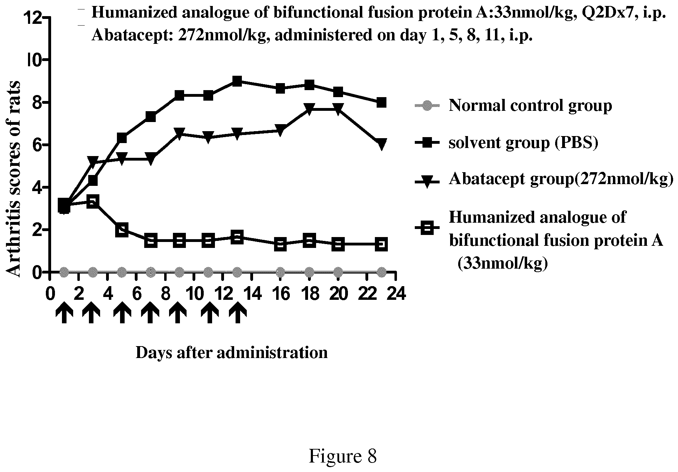

The experimental results are shown in Figure 8. The rats in the normal control group do not produce inflammatory responses. The CIA model rats in the solvent group produce obvious inflammatory responses. The arthritis scores of the CIA model rats in the group administered with the humanized analogue of the bifunctional fusion protein A are significantly inhibited, showing a better amelioration effect of inflammation than that in the group administered with Abatacept.

## Claims

1. A bifunctional fusion protein, comprising the extracellular domain of CTLA4 and an anti-IL-17 antibody, preferably, IL-17 is IL-17a, wherein the extracellular domain of the CTLA4 molecule comprises an amino acid sequence as set forth in SEQ ID NO.2 or an amino acid sequence having at least 70% identity thereto and having the same function.

2. The bifunctional fusion protein of claim 1, further comprising a linker, wherein the extracellular domain of a CTLA4 molecule is linked to N-terminal or C-terminal of the functional fragment which neutralizes the activity of IL-17 via a linker, preferably the linker is 1-25 amino acids in length, more preferably, the linker is an amino acid sequence as set forth in SEQ ID NO.3.

3. The bifunctional fusion protein of claim 1 or 2, wherein the functional fragment which neutralizes the activity of IL-17 is an anti-IL-17 antibody or a functional fragment thereof, a chimeric antibody, a humanized antibody, a full humanized antibody, a single-chain antibody or a bispecific antibody, preferably the extracellular domain of a CTLA4 molecule is linked to N-terminal of a heavy chain or a light chain of an anti-IL-17 antibody via a linker, preferably the anti-IL-17 antibody is an antibody selected from the group consisting of IgG, IgA, IgD, IgE, IgM antibody, and a hybrid thereof, more preferably the anti-IL-17 antibody is an IgG antibody.

4. The bifunctional fusion protein of any one of claims 1-3, wherein the heavy chain sequence of the anti-IL-17 antibody comprises an amino acid sequence as set forth in SEQ ID NO.28, or an amino acid sequence having at least 70% identity thereto and having the same function, and wherein the light chain sequence of the anti-IL-17 antibody comprises an amino acid sequence as set forth in SEQ ID NO.27, or an amino acid sequence having at least 70% identity thereto and having the same function; and/or the extracellular domain of the CTLA4 molecule as set forth in SEQ ID NO.2 is linked to N-terminal of the heavy chain sequence of an anti-IL-17 antibody as set forth in SEQ ID NO.4 or 12 or to N-terminal of the light chain sequence of an anti-IL-17 antibody as set forth in SEQ ID NO.5 or 13 via a linker as set forth in SEQ ID NO.3; and/or the extracellular domain of the CTLA4 molecule as set forth in SEQ ID NO.2 is linked to C-terminal of the heavy chain sequence of an anti-IL-17 antibody as set forth in SEQ ID NO.4 or 12 or to C-terminal of the light chain sequence of an anti-IL-17 antibody as set forth in SEQ ID NO.5 or 13 via a linker as set forth in SEQ ID NO.3.

5. The bifunctional fusion protein of any one of claims 1-4, wherein the amino acid sequence thereof is selected from the group consisting of the following sequence combinations:
a) sequences as set forth in SEQ ID NOs. 5 and 6, SEQ ID NOs. 13 and 14, SEQ ID NOs. 4 and 19, SEQ ID NOs. 24 and 25 and SEQ ID NOs. 26 and 27;
b) amino acid sequences derived from the sequences as set forth in SEQ ID NOs. 5 and 6, SEQ ID NOs. 13 and 14, SEQ ID NOs. 4 and 19, SEQ ID NOs. 24 and 25 and SEQ ID NOs. 26 and 27 through substitution, deletion or addition of one or more amino acid residues, and having an activity for blocking B7 and IL-17A signal pathways simultaneously; or
c) amino acid sequences having at least 70% identity to the sequences as set forth in SEQ ID NOs. 5 and 6, SEQ ID NOs. 13 and 14, SEQ ID NOs. 4 and 19, SEQ ID NOs. 24 and 25 and SEQ ID NOs. 26 and 27, and having an activity for blocking B7 and IL-17A signal pathways simultaneously.

6. A gene encoding the bifunctional fusion protein according to any one of claims 1-5.

7. The gene of claim 6, wherein the gene comprises nucleotide sequences as set forth in SEQ ID NOs.7 and 8, or SEQ ID NOs. 15 and 16.

8. A recombinant vector, comprising the gene according to claim 6 or 7 operably linked therein.

9. A host cell, comprising the recombinant vector according to claim 8.

10. A method for preparing the bifunctional fusion protein according to any one of claims 1-5, comprising the following steps:
(1) operably linking the gene according to claim 6 or 7 into an eukaryotic expression vector, and transfecting the same to a host cell for expression or transfecting the recombinant vector according to claim 8 to a host cell for expression; and
(2) purifying the bifunctional fusion protein; preferably, the eukaryotic expression vector is XOGC, and preferably, the host cell is a HEK293-T or a CHO cell.

11. A pharmaceutical composition, comprising the bifunctional fusion protein according to any one of claims 1-5 or a bifunctional fusion protein produced according to the method of claim 10.

12. The bifunctional fusion protein according to any one of claims 1-5, or a bifunctional fusion protein produced according to the method of claim 10 or the pharmaceutical composition according to claim 11 for use in the prevention, treatment or amelioration of a disease.

13. The bifunctional fusion protein or the pharmaceutical composition according to claim 12, wherein the disease is selected from a group consisting of rheumatoid arthritis, psoriasis, psoriatic arthritis, ankylosing spondylitis, Crohn's disease, systemic lupus erythematosus, lupus nephritis, multiple sclerosis, autoimmune encephalomyelitis, glomerulonephritis, idiopathic thrombocytopenic purpura, primary sjogren's syndrome, gout or organ transplantation.

14. A kit, comprising the bifunctional fusion protein according to any one of claims 1-5, the gene according to claim 6 or 7, the recombinant vector according to claim 8 or the host cell according to claim 9.

## Patentansprüche

1. Bifunktionelles Fusionsprotein, umfassend die extrazelluläre Domäne von CTLA4 und einen Anti-IL-17-Antikörper, wobei IL-17 vorzugsweise IL-17a ist, wobei die extrazelluläre Domäne des CTLA4-Moleküls eine Aminosäuresequenz, wie sie in SEQ ID Nr. 2 angegeben ist, oder eine Aminosäuresequenz umfasst, die wenigstens 70 % Identität damit aufweist und die gleiche Funktion hat.

2. Bifunktionelles Fusionsprotein nach Anspruch 1, ferner umfassend einen Linker, wobei die extrazelluläre Domäne eines CTLA4-Moleküls über einen Linker mit dem N-Terminus oder dem C-Terminus des funktionellen Fragments verknüpft ist, das die Aktivität von IL-17 neutralisiert, wobei der Linker vorzugsweise 1 bis 25 Aminosäuren lang ist und wobei der Linker stärker bevorzugt eine Aminosäuresequenz ist, wie sie in SEQ ID Nr. 3 angegeben ist.

3. Bifunktionelles Fusionsprotein nach Anspruch 1 oder 2, wobei das funktionelle Fragment, das die Aktivität von IL-17 neutralisiert, ein Anti-IL-17-Antikörper oder ein funktionelles Fragment davon, ein chimärer Antikörper, ein humanisierter Antikörper, ein vollständig humanisierter Antikörper, ein Einzelkettenantikörper oder ein bispezifischer Antikörper ist, wobei die extrazelluläre Domäne eines CTLA4-Moleküls vorzugsweise über einen Linker mit dem N-Terminus einer schweren Kette oder einer leichten Kette eines Anti-IL-17-Antikörpers verknüpft ist, wobei der Anti-IL-17-Antikörper vorzugsweise ein Antikörper ist, der aus der Gruppe ausgewählt ist, die aus einem IgG-, einem IgA-, einem IgD-, einem IgE-, einem IgM-Antikörper und einem Hybrid davon besteht, wobei der Anti-IL-17-Antikörper stärker bevorzugt ein IgG-Antikörper ist.

4. Bifunktionelles Fusionsprotein nach einem der Ansprüche 1 bis 3, wobei die Schwerkettensequenz des Anti-IL-17-Antikörpers eine Aminosäuresequenz, wie sie in SEQ ID Nr. 28 angegeben ist, oder eine Aminosäuresequenz umfasst, die wenigstens 70 % Identität damit aufweist und die gleiche Funktion hat, und wobei die Leichtkettensequenz des Anti-IL-17-Antikörpers eine Aminosäuresequenz, wie sie in SEQ ID Nr. 27 angegeben ist, oder eine Aminosäuresequenz umfasst, die wenigstens 70 % Identität damit aufweist und die gleiche Funktion hat; und/oder wobei die extrazelluläre Domäne des CTLA4-Moleküls, wie sie in SEQ ID Nr. 2 angegeben ist, mit dem N-Terminus der Schwerkettensequenz eines Anti-IL-17-Antikörpers, wie sie in SEQ ID Nr. 4 oder 12 angegeben ist, oder mit dem N-Terminus der Leichtkettensequenz eines Anti-IL-17-Antikörpers, wie sie in SEQ ID Nr. 5 oder 13 angegeben ist, über einen Linker, wie er in SEQ ID Nr. 3 angegeben ist, verknüpft ist; und/oder wobei die extrazelluläre Domäne des CTLA4-Moleküls, wie sie in SEQ ID Nr. 2 angegeben ist, mit dem C-Terminus der Schwerkettensequenz eines Anti-IL-17-Antikörpers, wie sie in SEQ ID Nr. 4 oder 12 angegeben ist, oder mit dem C-Terminus der Leichtkettensequenz eines Anti-IL-17-Antikörpers, wie sie in SEQ ID Nr. 5 oder 13 angegeben ist, über einen Linker, wie er in SEQ ID Nr. 3 angegeben ist, verknüpft ist.

5. Bifunktionelles Fusionsprotein nach einem der Ansprüche 1 bis 4, wobei die Aminosäuresequenz davon aus der Gruppe ausgewählt ist, die aus den folgenden Sequenzkombinationen besteht:
(a) Sequenzen, wie sie in SEQ ID Nr. 5 und 6, SEQ ID Nr. 13 und 14, SEQ ID Nr. 4 und 19, SEQ ID Nr. 24 und 25 und SEQ ID Nr. 26 und 27 angegeben sind;
(b) Aminosäuresequenzen, die von den Sequenzen, wie sie in SEQ ID Nr. 5 und 6, SEQ ID Nr. 13 und 14, SEQ ID Nr. 4 und 19, SEQ ID Nr. 24 und 25 und SEQ ID Nr. 26 und 27 angegeben sind, durch Substitution, Deletion oder Hinzufügen von einem oder mehreren Aminosäureresten abgeleitet sind und die eine Aktivität zur gleichzeitigen Blockierung der B7- und IL-17A-Signalwege aufweisen; oder
(c) Aminosäuresequenzen, die wenigstens 70 % Identität mit den Sequenzen aufweisen, wie sie in SEQ ID Nr. 5 und 6, SEQ ID Nr. 13 und 14, SEQ ID Nr. 4 und 19, SEQ ID Nr. 24 und 25 und SEQ ID Nr. 26 und 27 angegeben sind, und die eine Aktivität zur gleichzeitigen Blockierung der B7- und IL-17A-Signalwege aufweisen.

6. Gen, das für das bifunktionelle Fusionsprotein nach einem der Ansprüche 1 bis 5 codiert.

7. Gen nach Anspruch 6, wobei das Gen Nukleotidsequenzen umfasst, wie sie in SEQ ID Nr. 7 und 8 oder SEQ ID Nr. 15 und 16 angegeben sind.

8. Rekombinanter Vektor, umfassend das wirkmäßig darin eingekoppelte Gen nach Anspruch 6 oder 7.

9. Wirtszelle, umfassend den rekombinanten Vektor nach Anspruch 8.

10. Verfahren zur Herstellung des bifunktionellen Fusionsproteins nach einem der Ansprüche 1 bis 5, umfassend die folgenden Schritte:
(1) wirkmäßiges Einkoppeln des Gens nach Anspruch 6 oder 7 in einen eukaryotischen Expressionsvektor und Transfizieren desselben in eine Wirtszelle zur Expression oder Transfektion des rekombinanten Vektors nach Anspruch 8 in eine Wirtszelle zur Expression; und
(2) Reinigen des bifunktionellen Fusionsproteins; wobei der eukaryotische Expressionsvektor vorzugsweise X0CG ist und wobei die Wirtszelle vorzugsweise eine HEK293-T- oder eine CHO-Zelle ist.

11. Pharmazeutische Zusammensetzung, umfassend das bifunktionelle Fusionsprotein nach einem der Ansprüche 1 bis 5 oder ein bifunktionelles Fusionsprotein, das nach dem Verfahren von Anspruch 10 hergestellt wurde.

12. Bifunktionelles Fusionsprotein nach einem der Ansprüche 1 bis 5 oder bifunktionelles Fusionsprotein, das nach dem Verfahren von Anspruch 10 hergestellt wurde, oder pharmazeutische Zusammensetzung nach Anspruch 11 zur Verwendung bei der Vorbeugung, Behandlung oder Linderung einer Krankheit.

13. Bifunktionelles Fusionsprotein oder pharmazeutische Zusammensetzung nach Anspruch 12, wobei die Krankheit aus einer Gruppe ausgewählt ist, die aus rheumatoider Arthritis, Psoriasis, psoriatischer Arthritis, Spondylitis ankylosans, Morbus Crohn, systemischem Lupus erythematodes, Lupus-Nephritis, multipler Sklerose, Autoimmun-Enzphalomyelitis, Glomerulonephritis, idiopathischer thrombozytopenischer Purpura, primärem Sjögren-Syndrom, Gicht oder Organtransplantation besteht.

14. Kit, umfassend das bifunktionelle Fusionsprotein nach einem der Ansprüche 1 bis 5, das Gen nach Anspruch 6 oder 7, den rekombinanten Vektor nach Anspruch 8 oder die Wirtszelle nach Anspruch 9.

## Revendications

1. Protéine de fusion bifonctionnelle, comprenant le domaine extracellulaire de CTLA4 et un anticorps anti-IL-17, de préférence, IL-17 est IL-17a, le domaine extracellulaire de la molécule CTLA4 comprenant une séquence d'acides aminés telle que présentée dans SEQ ID n° 2 ou une séquence d'acides aminés ayant au moins 70 % d'identité à celle-ci et ayant la même fonction.

2. Protéine de fusion bifonctionnelle selon la revendication 1, comprenant en outre un lieur, le domaine extracellulaire d'une molécule de CTLA4 étant liée à une extrémité N-terminale ou C-terminale du fragment fonctionnel qui neutralise l'activité d'IL-17 via un lieur, de préférence le lieur est de 1 à 25 acides aminés de long, davantage de préférence, le lieur est une séquence d'acides aminés telle que présentée dans SEQ ID n° 3.

3. Protéine de fusion bifonctionnelle selon la revendication 1 ou 2, dans laquelle le fragment fonctionnel qui neutralise l'activité d'IL-17 est un anticorps anti-IL-17 ou un de ses fragments fonctionnels, un anticorps chimérique, un anticorps humanisé, un anticorps complètement humanisé, un anticorps monocaténaire ou un anticorps bispécifique, de préférence le domaine extracellulaire d'une molécule de CTLA4 est lié à l'extrémité N-terminale d'une chaîne lourde ou d'une chaîne légère d'un anticorps anti-IL-17 via un lieur, de préférence l'anticorps anti-IL-17 est choisi dans le groupe constitué par l'anticorps IgG, IgA, IgD, IgE, IgM et un de leurs hybrides, davantage de préférence l'anticorps anti-IL-17 est un anticorps IgG.

4. Protéine de fusion bifonctionnelle selon l'une quelconque des revendications 1 à 3, dans laquelle la séquence de chaîne lourde de l'anticorps anti-IL-17 comprend une séquence d'acides aminés telle que présentée dans SEQ ID n° 28, ou une séquence d'acides aminés ayant au moins 70 % d'identité à celle-ci et ayant la même fonction, et la séquence de chaîne légère de l'anticorps anti-IL-17 comprenant une séquence d'acides aminés telle que présentée dans SEQ ID n° 27, ou une séquence d'acides aminés ayant au moins 70 % d'identité à celle-ci et ayant la même fonction ; et/ou le domaine extracellulaire de la molécule CTLA4 tel que présenté dans SEQ ID n° 2 est lié à l'extrémité N-terminale de la séquence de chaîne lourde d'un anticorps anti-IL-17 tel que présenté dans SEQ ID n° 4 ou 12 ou à l'extrémité N-terminale de la séquence de chaîne légère d'un anticorps anti-IL-17 telle que présentée dans SEQ ID n° 5 ou 13 via un lieur tel que présenté dans SEQ ID n° 3 ; et/ou le domaine extracellulaire de la molécule CTLA4 tel que présenté dans SEQ ID n° 2 est lié à l'extrémité C-terminale de la séquence de chaîne lourde d'un anticorps anti-IL-17 tel que présenté dans SEQ ID n° 4 ou 12 ou à l'extrémité C-terminale de la séquence de chaîne légère d'un anticorps anti-IL-17 tel que présenté dans SEQ ID n° 4 ou 12 ou l'extrémité C-terminale de la séquence de chaîne légère d'un anticorps anti-IL-17 telle que présentée dans SEQ ID n° 5 ou 13 via un lieur tel que présenté dans SEQ ID n° 3.

5. Protéine de fusion bifonctionnelle selon l'une quelconques revendications 1 à 4, dans laquelle sa séquence d'acides aminés est choisie dans le groupe constitué par les combinaisons de séquences suivantes :
a) les séquences telles que présentées dans SEQ ID n° 5 et 6, SEQ ID n° 13 et 14, SEQ ID n° 4 et 19, SEQ ID n° 24 et 25 et SEQ ID n° 26 et 27 ;
b) les séquences d'acides aminés dérivées des séquences présentées dans SEQ ID n° 5 et 6, SEQ n° 13 et 14, SEQ n° 4 et 19, SEQ n° 24 et 25 et SEQ n° 26 et 27 par substitution, délétion ou addition d'un ou de plusieurs résidus d'acide aminé, et ayant une activité pour bloquer les voies de signalisation de B7 et IL-17A simultanément ; ou
c) les séquences d'acides aminés ayant au moins 70 % d'identité aux séquences telles que présentées dans SEQ n° 5 et 6, SEQ n° 13 et 14, SEQ n° 4 et 19, SEQ n° 24 et 25 et SEQ n° 26 et 27, et ayant une activité pour bloquer les voies de signalisation de B7 et IL-17A simultanément.

6. Gène codant pour la protéine de fusion bifonctionnelle selon l'une quelconque des revendications 1 à 5.

7. Gène selon la revendication 6, dans lequel le gène comprend les séquences nucléotidiques telles que présentées dans SEQ ID n° 7 et 8 ou SEQ ID n° 15 et 16.

8. Vecteur recombinant, comprenant le gène selon la revendication 6 ou 7 lié opérationnellement à l'intérieur.

9. Cellule hôte, comprenant le vecteur recombinant selon la revendication 8.

10. Procédé de préparation de la protéine de fusion bifonctionnelle selon l'une quelconque des revendications 1 à 5, comprenant les étapes suivantes consistant à :
(1) lier opérationnellement le gène selon la revendication 6 ou 7 dans un vecteur d'expression eucaryote, et transfecter le même à une cellule hôte pour l'expression ou transfecter le vecteur recombinant selon la revendication 8 à une cellule hôte pour l'expression ; et
(2) purifier la protéine de fusion bifonctionnelle ;
de préférence, le vecteur d'expression eucaryote est XOGC et, de préférence, la cellule hôte est une cellule HEK293-T ou CHO.

11. Composition pharmaceutique, comprenant la protéine de fusion bifonctionnelle selon l'une quelconque des revendications 1 à 5 ou une protéine de fusion bifonctionnelle produite selon le procédé de la revendication 10.

12. Protéine de fusion bifonctionnelle selon l'une quelconque des revendications 1 à 5, ou protéine de fusion bifonctionnelle produite selon le procédé de la revendication 10 ou composition pharmaceutique selon la revendication 11 destinée à être utilisée dans la prévention, le traitement ou l'amélioration d'une maladie.

13. Protéine de fusion bifonctionnelle ou composition pharmaceutique selon la revendication 12, dans laquelle la maladie est choisie dans un groupe constitué par la polyarthrite rhumatoïde, le psoriasis, l'arthrite psoriatique, la spondylite ankylosante, la maladie de Crohn, le lupus érythémateux systémique, la néphrite du lupus, la sclérose en plaques, l'encéphalomyélite autoimmune, la glomérulonéphrite, le purpura thrombocytopénique idiopathique, le syndrome de sjogren primaire, la goutte ou la greffe d'organe.

14. Kit, comprenant la protéine de fusion bifonctionnelle selon l'une quelconque des revendications 1 à 5, le gène selon la revendication 6 ou 7, le vecteur recombinant selon la revendication 8 ou la cellule hôte selon la revendication 9.


### SEQUENCE LISTING

<110> BEIJING HANMI PHARMACEUTICAL CO., LTD.
<120> BIFUNCTIONAL FUSION PROTEIN, PREPARATION METHOD THEREFOR, AND USE THEREOF
<130> 135189CN01
<160> 28
<170> PatentIn version 3.3
<210> 1
   <211> 25
   <212> PRT
   <213> Artificial
<220>
   <223> Signal peptide
<400> 1
   Met Gly Val Leu Leu Thr Gln Arg Thr Leu Leu Ser Leu Val Leu Ala
   1               5                   10                  15
   Leu Leu Phe Pro Ser Met Ala Ser Met
               20                  25
<210> 2
   <211> 125
   <212> PRT
   <213> Human
<400> 2
   Ala Met His Val Ala Gln Pro Ala Val Val Leu Ala Ser Ser Arg Gly
   1               5                   10                  15
   Ile Ala Ser Phe Val Cys Glu Tyr Ala Ser Pro Gly Lys Ala Thr Glu
               20                  25                  30
   Val Arg Val Thr Val Leu Arg Gln Ala Asp Ser Gln Val Thr Glu Val
           35                  40                  45
   Cys Ala Ala Thr Tyr Met Met Gly Asn Glu Leu Thr Phe Leu Asp Asp
       50                  55                  60
   Ser Ile Cys Thr Gly Thr Ser Ser Gly Asn Gln Val Asn Leu Thr Ile
   65                  70                  75                  80
   Gln Gly Leu Arg Ala Met Asp Thr Gly Leu Tyr Ile Cys Lys Val Glu
                   85                  90                  95
   Leu Met Tyr Pro Pro Pro Tyr Tyr Leu Gly Ile Gly Asn Gly Thr Gln
                   100                 105                 110
   Ile Tyr Val Ile Asp Pro Glu Pro Cys Pro Asp Ser Asp
           115                 120                 125
<210> 3
   <211> 25
   <212> PRT
   <213> Artificial
<220>
   <223> Linker
<400> 3
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 4
   <211> 452
   <212> PRT
   <213> Artificial
<220>
   <223> anti-IL-17A mAb hc-A
<400> 4
   Gln Val Gln Leu Lys Glu Ser Gly Pro Gly Leu Val Gln Pro Ser Gln
   1               5                   10                  15
   Thr Leu Ser Leu Thr Cys Thr Val Ser Gly Phe Ser Leu Thr Asn Tyr
               20                  25                  30
   Tyr Val His Trp Val Arg Gln Pro Pro Gly Lys Gly Leu Glu Trp Met
           35                  40                  45
   Gly Gly Val Trp Asn Asp Gly Asp Thr Ser Tyr Asn Ser Val Leu Arg
       50                  55                  60
   Ser Arg Leu Ser Ile Thr Arg Asp Thr Ser Lys Ser Gln Val Leu Leu
   65                  70                  75                  80
   Lys Met Ser Ser Leu Gln Thr Glu Asp Thr Ala Thr Tyr Tyr Cys Ala
                   85                  90                  95
   Arg Glu Gly Arg Glu Gly Phe Val Gly Tyr Tyr Val Met Asp Ala Trp
               100                 105                 110
   Gly Pro Gly Ala Ser Val Thr Val Ser Ser Ala Lys Thr Thr Ala Pro
           115                 120                 125
   Ser Val Tyr Pro Leu Ala Pro Val Cys Gly Asp Thr Thr Gly Ser Ser
       130                 135                 140
   Val Thr Leu Gly Cys Leu Val Lys Gly Tyr Phe Pro Glu Pro Val Thr
   145                 150                 155                 160
   Leu Thr Trp Asn Ser Gly Ser Leu Ser Ser Gly Val His Thr Phe Pro
                   165                 170                 175
   Ala Val Leu Gln Ser Asp Leu Tyr Thr Leu Ser Ser Ser Val Thr Val
               180                 185                 190
   Thr Ser Ser Thr Trp Pro Ser Gln Ser Ile Thr Cys Asn Val Ala His
           195                 200                 205
   Pro Ala Ser Ser Thr Lys Val Asp Lys Lys Ile Glu Pro Arg Gly Pro
       210                 215                 220
   Thr Ile Lys Pro Cys Pro Pro Cys Lys Cys Pro Ala Pro Asn Leu Leu
   225                 230                 235                 240
   Gly Gly Pro Ser Val Phe Ile Phe Pro Pro Lys Ile Lys Asp Val Leu
                   245                 250                 255
   Met Ile Ser Leu Ser Pro Ile Val Thr Cys Val Val Val Asp Val Ser
               260                 265                 270
   Glu Asp Asp Pro Asp Val Gln Ile Ser Trp Phe Val Asn Asn Val Glu
           275                 280                 285
   Val His Thr Ala Gln Thr Gln Thr His Arg Glu Asp Tyr Asn Ser Thr
       290                 295                 300
   Leu Arg Val Val Ser Ala Leu Pro Ile Gln His Gln Asp Trp Met Ser
   305                 310                 315                 320
   Gly Lys Glu Phe Lys Cys Lys Val Asn Asn Lys Asp Leu Pro Ala Pro
                   325                 330                 335
   Ile Glu Arg Thr Ile Ser Lys Pro Lys Gly Ser Val Arg Ala Pro Gln
               340                 345                 350
   Val Tyr Val Leu Pro Pro Pro Glu Glu Glu Met Thr Lys Lys Gln Val
           355                 360                 365
   Thr Leu Thr Cys Met Val Thr Asp Phe Met Pro Glu Asp Ile Tyr Val
       370                 375                 380
   Glu Trp Thr Asn Asn Gly Lys Thr Glu Leu Asn Tyr Lys Asn Thr Glu
   385                 390                 395                 400
   Pro Val Leu Asp Ser Asp Gly Ser Tyr Phe Met Tyr Ser Lys Leu Arg
                   405                 410                 415
   Val Glu Lys Lys Asn Trp Val Glu Arg Asn Ser Tyr Ser Cys Ser Val
               420                 425                 430
   Val His Glu Gly Leu His Asn His His Thr Thr Lys Ser Phe Ser Arg
           435                 440                 445
   Thr Pro Gly Lys
       450
<210> 5
   <211> 214
   <212> PRT
   <213> Artificial
<220>
   <223> anti-IL-17A mAb lc-A
<400> 5
   Asp Ile Gln Met Thr Gln Ser Pro Ser Phe Leu Ser Ala Ser Val Gly
   1               5                   10                  15
   Glu Arg Val Thr Leu Ser Cys Lys Ala Ser Gln Asn Ile Asn Lys Tyr
               20                  25                  30
   Leu Asp Trp Phe Gln Gln Lys Leu Gly Glu Ala Pro Lys Leu Leu Ile
           35                  40                  45
   Tyr Asn Ala Asp Asn Leu His Thr Gly Ile Pro Ser Arg Phe Ser Gly
       50                  55                  60
   Ser Gly Ser Phe Ser Asp Phe Ile Leu Thr Ile Ser Ser Leu Gln Pro
   65                  70                  75                  80
   Glu Asp Asp Ala Thr Tyr Phe Cys Leu Gln Arg Glu Ser Trp Pro Tyr
                   85                  90                  95
   Thr Phe Gly Ala Gly Thr Lys Leu Glu Leu Lys Arg Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 6
   <211> 602
   <212> PRT
   <213> Artificial
<220>
   <223> Human CTLA4 ECD -linker- anti-IL-17A mAb hc-A
<400> 6
   Ala Met His Val Ala Gln Pro Ala Val Val Leu Ala Ser Ser Arg Gly
   1               5                   10                  15
   Ile Ala Ser Phe Val Cys Glu Tyr Ala Ser Pro Gly Lys Ala Thr Glu
               20                  25                  30
   Val Arg Val Thr Val Leu Arg Gln Ala Asp Ser Gln Val Thr Glu Val
           35                  40                  45
   Cys Ala Ala Thr Tyr Met Met Gly Asn Glu Leu Thr Phe Leu Asp Asp
       50                  55                  60
   Ser Ile Cys Thr Gly Thr Ser Ser Gly Asn Gln Val Asn Leu Thr Ile
   65                  70                  75                  80
   Gln Gly Leu Arg Ala Met Asp Thr Gly Leu Tyr Ile Cys Lys Val Glu
                   85                  90                  95
   Leu Met Tyr Pro Pro Pro Tyr Tyr Leu Gly Ile Gly Asn Gly Thr Gln
   100 105 110
   Ile Tyr Val Ile Asp Pro Glu Pro Cys Pro Asp Ser Asp Gly Gly Gly
   115 120 125
   Gly Ser Gly Gly Gly Gly Ser Gly Gly Gly Gly Ser Gly Gly Gly Gly
   130 135 140
   Ser Gly Gly Gly Gly Ser Gln Val Gln Leu Lys Glu Ser Gly Pro Gly
   145 150 155 160
   Leu Val Gln Pro Ser Gln Thr Leu Ser Leu Thr Cys Thr Val Ser Gly
   165 170 175
   Phe Ser Leu Thr Asn Tyr Tyr Val His Trp Val Arg Gln Pro Pro Gly
   180 185 190
   Lys Gly Leu Glu Trp Met Gly Gly Val Trp Asn Asp Gly Asp Thr Ser
   195 200 205
   Tyr Asn Ser Val Leu Arg Ser Arg Leu Ser Ile Thr Arg Asp Thr Ser
   210 215 220
   Lys Ser Gln Val Leu Leu Lys Met Ser Ser Leu Gln Thr Glu Asp Thr
   225 230 235 240
   Ala Thr Tyr Tyr Cys Ala Arg Glu Gly Arg Glu Gly Phe Val Gly Tyr
   245 250 255
   Tyr Val Met Asp Ala Trp Gly Pro Gly Ala Ser Val Thr Val Ser Ser
   260 265 270
   Ala Lys Thr Thr Ala Pro Ser Val Tyr Pro Leu Ala Pro Val Cys Gly
   275 280 285
   Asp Thr Thr Gly Ser Ser Val Thr Leu Gly Cys Leu Val Lys Gly Tyr
   290 295 300
   Phe Pro Glu Pro Val Thr Leu Thr Trp Asn Ser Gly Ser Leu Ser Ser
   305 310 315 320
   Gly Val His Thr Phe Pro Ala Val Leu Gln Ser Asp Leu Tyr Thr Leu
   325 330 335
   Ser Ser Ser Val Thr Val Thr Ser Ser Thr Trp Pro Ser Gln Ser Ile
   340 345 350
   Thr Cys Asn Val Ala His Pro Ala Ser Ser Thr Lys Val Asp Lys Lys
           355                 360                 365
   Ile Glu Pro Arg Gly Pro Thr Ile Lys Pro Cys Pro Pro Cys Lys Cys
       370                 375                 380
   Pro Ala Pro Asn Leu Leu Gly Gly Pro Ser Val Phe Ile Phe Pro Pro
   385                 390                 395                 400
   Lys Ile Lys Asp Val Leu Met Ile Ser Leu Ser Pro Ile Val Thr Cys
                   405                 410                 415
   Val Val Val Asp Val Ser Glu Asp Asp Pro Asp Val Gln Ile Ser Trp
               420                 425                 430
   Phe Val Asn Asn Val Glu Val His Thr Ala Gln Thr Gln Thr His Arg
           435                 440                 445
   Glu Asp Tyr Asn Ser Thr Leu Arg Val Val Ser Ala Leu Pro Ile Gln
       450                 455                 460
   His Gln Asp Trp Met Ser Gly Lys Glu Phe Lys Cys Lys Val Asn Asn
   465                 470                 475                 480
   Lys Asp Leu Pro Ala Pro Ile Glu Arg Thr Ile Ser Lys Pro Lys Gly
                   485                 490                 495
   Ser Val Arg Ala Pro Gln Val Tyr Val Leu Pro Pro Pro Glu Glu Glu
               500                 505                 510
   Met Thr Lys Lys Gln Val Thr Leu Thr Cys Met Val Thr Asp Phe Met
           515                 520                 525
   Pro Glu Asp Ile Tyr Val Glu Trp Thr Asn Asn Gly Lys Thr Glu Leu
       530                 535                 540
   Asn Tyr Lys Asn Thr Glu Pro Val Leu Asp Ser Asp Gly Ser Tyr Phe
   545                 550                 555                 560
   Met Tyr Ser Lys Leu Arg Val Glu Lys Lys Asn Trp Val Glu Arg Asn
                   565                 570                 575
   Ser Tyr Ser Cys Ser Val Val His Glu Gly Leu His Asn His His Thr
               580                 585                 590
   Thr Lys Ser Phe Ser Arg Thr Pro Gly Lys
           595                 600
<210> 7
   <211> 1806
   <212> DNA
   <213> Artificial
<220>
   <223> Human CTLA4 ECD - linker - anti-IL-17A mAb hc-A encoding nucleotide
   sequence
<400> 7
   gctatgcacg tcgctcagcc agcagtggtc ctggcatcca gcagaggcat cgccagtttc     60
   gtgtgcgagt acgcttcacc cggaaaggca acagaagtca gagtgactgt cctgcgacag    120
   gcagacagcc aggtgaccga ggtctgcgcc gctacatata tgatgggcaa cgaactgaca    180
   tttctggacg attctatctg tactggcacc tctagtggaa accaagtgaa tctgactatc    240
   cagggcctgc gcgctatgga taccggactg tacatttgca aggtggagct gatgtatccc    300
   cctccatact atctggggat tggtaatggc acccaaatat acgtgattga cccagagccc    360
   tgtcctgact ctgatggagg tggaggatcc ggaggtggag gaagcggggg tggcggatct    420
   ggaggaggag ggtctggcgg cggaggaagt caggtgcaac tgaaggagag cggccccgga    480
   ctggtgcaac cttcccaaac actcagcctc acctgtaccg tctccggatt ctccctgacc    540
   aactattacg tccactgggt cagacagcct cccggcaagg gactggagtg gatgggaggc    600
   gtgtggaatg acggcgacac ctcctacaac agcgtcctca ggagcagact gagcattacc    660
   agagacacca gcaagagcca ggtcctcctc aagatgtcct ccctccagac agaagacacc    720
   gccacctatt actgcgccag ggaaggcagg gaaggcttcg tcggctacta tgtgatggat    780
   gcttggggcc ctggagctag cgtgacagtg agctccgcaa agaccacagc accaagcgtg    840
   tacccactgg cacctgtctg cggcgacact accggatcca gtgtgacact gggatgtctg    900
   gtcaagggct atttccccga gcctgtgact ctgacctgga atagcgggtc cctgtcaagc    960
   ggagtgcaca catttcccgc tgtcctgcag tccgacctgt atactctgtc ctctagtgtg   1020
   acagtcactt caagcacctg gccctctcag agtattacat gcaacgtggc ccatcctgct   1080
   tcctctacta aggtggataa gaaaatcgaa ccaagaggcc ccaccattaa gccttgtcca   1140
   ccctgcaaat gtccagcacc aaatctgctg ggaggaccaa gcgtgttcat ctttcctcca   1200
   aagatcaagg acgtgctgat gatctcactg agcccaattg tgacctgcgt ggtcgtggac   1260
   gtgagcgagg acgatcccga tgtgcagatc agctggttcg tcaacaatgt ggaagtccac   1320
   accgcccaga cccagacaca tagagaggat tacaactcta cactgagggt cgtgagtgct   1380
   ctgcccattc agcatcagga ctggatgagc gggaaggagt tcaagtgcaa agtgaacaac   1440
   aaggacctgc ctgctccaat cgagaggacc attagcaagc ctaaaggcag cgtgcgggca   1500
   ccacaggtgt atgtcctgcc ccctccagag gaagagatga ccaagaaaca agtgactctg   1560
   acctgtatgg tcacagactt catgcccgaa gatatctacg tggagtggac aaacaacgga   1620
   aagactgaac tgaactacaa gaacaccgag cctgtgctgg actccgatgg ctcttacttt   1680
   atgtatagca agctgcgcgt ggaaaagaaa aactgggtcg agcgaaattc ctactcttgt   1740
   agtgtggtcc acgaagggct gcataatcat catactacca agtctttttc cagaacccca   1800
   ggcaaa                                                              1806
<210> 8
   <211> 642
   <212> DNA
   <213> Artificial
<220>
   <223> anti-IL-17A mAb lc-A encoding nucleotide sequence
<400> 8
   gacatccaga tgacacagtc ccccagcttc ctgagcgcta gcgtgggaga gagggtgacc 60
   ctgagctgca aggccagcca gaacatcaac aagtacctgg actggttcca gcagaagctg 120
   ggcgaagccc ccaagctgct gatctacaac gccgacaacc tccataccgg cattcccagc 180
   aggttcagcg gctccggctc cttcagcgac ttcatcctga ccatcagcag cctgcagccc 240
   gaggatgacg ccacctactt ctgcctgcag agggaatcct ggccttacac cttcggcgcc 300
   ggcaccaagc tggaactgaa gagagctgac gctgccccta ccgtctccat ctttcccccc 360
   tccagcgagc aactcacaag cggcggcgct tccgtcgtgt gcttcctgaa caacttctac 420
   cccaaggaca tcaacgtgaa gtggaagatc gacggcagcg aaaggcagaa cggcgtcctg 480
   aacagctgga ccgaccagga ctccaaggac tccacctact ccatgtcctc caccctcaca 540
   ctgaccaagg atgagtacga gaggcacaac agctacacct gcgaggccac acacaaaaca 600
   tccaccagcc ccatcgtgaa gtccttcaac cggaatgagt gc 642
<210> 9
   <211> 27
   <212> DNA
   <213> Artificial
<220>
   <223> Primer
<400> 9
   cccaagcttg ccaccatggg ggtcctg 27
<210> 10
   <211> 27
   <212> DNA
   <213> Artificial
<220>
   <223> Primer
<400> 10
   ccggaattct catttgcctg gggttct 27
<210> 11
   <211> 24
   <212> DNA
   <213> Artificial
<220>
   <223> Primer
<400> 11
   ccggaattct cagcactcat tccg 24
<210> 12
   <211> 454
   <212> PRT
   <213> Artificial
<220>
   <223> anti-IL-17A mAb hc-B
<400> 12
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Phe Ser Leu Pro Ser His
               20                  25                  30
   Ser Val Ser Trp Ile Arg Gln Pro Pro Gly Lys Gly Leu Glu Trp Ile
           35                  40                  45
   Gly Ile Ile Trp Asn Gln Gly Gly Thr Asp Tyr Asn Ser Ala Phe Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Asn Ala Tyr Ile Thr Asp Tyr Tyr Tyr Glu Asn Tyr Phe Met Asp
               100                 105                 110
   Ala Trp Gly Gln Gly Thr Leu Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Lys Val Glu Pro
       210                 215                 220
   Lys Ser Cys Asp Lys Thr His Thr Cys Pro Pro Cys Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Asp Glu Leu Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
   405 410 415
   Leu Thr Val Asp Lys Ser Arg Trp Gln Gln Gly Asn Val Phe Ser Cys
   420 425 430
   Ser Val Met His Glu Ala Leu His Asn His Tyr Thr Gln Lys Ser Leu
   435 440 445
   Ser Leu Ser Pro Gly Lys
   450
<210> 13
   <211> 220
   <212> PRT
   <213> Artificial
<220>
   <223> anti-IL-17A mAb lc-B
<400> 13
   Asp Ile Val Met Thr Gln Ser Pro Leu Ser Leu Pro Val Thr Pro Gly
   1               5                   10                  15
   Glu Pro Ala Ser Ile Ser Cys Lys Ser Ser Gln Ser Leu Leu Phe Ser
               20                  25                  30
   Glu Asn Gln Lys Asn Tyr Leu Ala Trp Tyr Leu Gln Lys Pro Gly Gln
           35                  40                  45
   Ser Pro Gln Leu Leu Ile Tyr Trp Thr Ser Thr Arg Gln Ser Gly Val
       50                  55                  60
   Pro Asp Arg Phe Ser Gly Ser Gly Ser Gly Thr Asp Phe Thr Leu Lys
   65                  70                  75                  80
   Ile Ser Arg Val Glu Ala Glu Asp Val Gly Val Tyr Tyr Cys Gln Gln
                   85                  90                  95
   Ser Tyr Tyr Thr Pro Tyr Thr Phe Gly Gln Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 14
   <211> 604
   <212> PRT
   <213> Artificial
<220>
   <223> Human CTLA4 ECD -linker- anti-IL-17A mAb hc-B
<400> 14
   Ala Met His Val Ala Gln Pro Ala Val Val Leu Ala Ser Ser Arg Gly
   1               5                   10                  15
   Ile Ala Ser Phe Val Cys Glu Tyr Ala Ser Pro Gly Lys Ala Thr Glu
               20                  25                  30
   Val Arg Val Thr Val Leu Arg Gln Ala Asp Ser Gln Val Thr Glu Val
           35                  40                  45
   Cys Ala Ala Thr Tyr Met Met Gly Asn Glu Leu Thr Phe Leu Asp Asp
       50                  55                  60
   Ser Ile Cys Thr Gly Thr Ser Ser Gly Asn Gln Val Asn Leu Thr Ile
   65                  70                  75                  80
   Gln Gly Leu Arg Ala Met Asp Thr Gly Leu Tyr Ile Cys Lys Val Glu
                   85                  90                  95
   Leu Met Tyr Pro Pro Pro Tyr Tyr Leu Gly Ile Gly Asn Gly Thr Gln
               100                 105                 110
   Ile Tyr Val Ile Asp Pro Glu Pro Cys Pro Asp Ser Asp Gly Gly Gly
           115                 120                 125
   Gly Ser Gly Gly Gly Gly Ser Gly Gly Gly Gly Ser Gly Gly Gly Gly
       130                 135                 140
   Ser Gly Gly Gly Gly Ser Gln Val Gln Leu Gln Glu Ser Gly Pro Gly
   145                 150                 155                 160
   Leu Val Lys Pro Ser Glu Thr Leu Ser Leu Thr Cys Thr Val Ser Gly
                   165                 170                 175
   Phe Ser Leu Pro Ser His Ser Val Ser Trp Ile Arg Gln Pro Pro Gly
               180                 185                 190
   Lys Gly Leu Glu Trp Ile Gly Ile Ile Trp Asn Gln Gly Gly Thr Asp
           195                 200                 205
   Tyr Asn Ser Ala Phe Lys Ser Arg Val Thr Ile Ser Val Asp Thr Ser
       210                 215                 220
   Lys Asn Gln Phe Ser Leu Lys Leu Ser Ser Val Thr Ala Ala Asp Thr
   225                 230                 235                 240
   Ala Val Tyr Tyr Cys Ala Arg Asn Ala Tyr Ile Thr Asp Tyr Tyr Tyr
                   245                 250                 255
   Glu Asn Tyr Phe Met Asp Ala Trp Gly Gln Gly Thr Leu Val Thr Val
               260                 265                 270
   Ser Ser Ala Ser Thr Lys Gly Pro Ser Val Phe Pro Leu Ala Pro Ser
           275                 280                 285
   Ser Lys Ser Thr Ser Gly Gly Thr Ala Ala Leu Gly Cys Leu Val Lys
       290                 295                 300
   Asp Tyr Phe Pro Glu Pro Val Thr Val Ser Trp Asn Ser Gly Ala Leu
   305                 310                 315                 320
   Thr Ser Gly Val His Thr Phe Pro Ala Val Leu Gln Ser Ser Gly Leu
                   325                 330                 335
   Tyr Ser Leu Ser Ser Val Val Thr Val Pro Ser Ser Ser Leu Gly Thr
               340                 345                 350
   Gln Thr Tyr Ile Cys Asn Val Asn His Lys Pro Ser Asn Thr Lys Val
           355                 360                 365
   Asp Lys Lys Val Glu Pro Lys Ser Cys Asp Lys Thr His Thr Cys Pro
       370                 375                 380
   Pro Cys Pro Ala Pro Glu Leu Leu Gly Gly Pro Ser Val Phe Leu Phe
   385                 390                 395                 400
   Pro Pro Lys Pro Lys Asp Thr Leu Met Ile Ser Arg Thr Pro Glu Val
                   405                 410                 415
   Thr Cys Val Val Val Asp Val Ser His Glu Asp Pro Glu Val Lys Phe
               420                 425                 430
   Asn Trp Tyr Val Asp Gly Val Glu Val His Asn Ala Lys Thr Lys Pro
           435                 440                 445
   Arg Glu Glu Gln Tyr Asn Ser Thr Tyr Arg Val Val Ser Val Leu Thr
       450                 455                 460
   Val Leu His Gln Asp Trp Leu Asn Gly Lys Glu Tyr Lys Cys Lys Val
   465                 470                 475                 480
   Ser Asn Lys Ala Leu Pro Ala Pro Ile Glu Lys Thr Ile Ser Lys Ala
                   485                 490                 495
   Lys Gly Gln Pro Arg Glu Pro Gln Val Tyr Thr Leu Pro Pro Ser Arg
               500                 505                 510
   Asp Glu Leu Thr Lys Asn Gln Val Ser Leu Thr Cys Leu Val Lys Gly
           515                 520                 525
   Phe Tyr Pro Ser Asp Ile Ala Val Glu Trp Glu Ser Asn Gly Gln Pro
       530                 535                 540
   Glu Asn Asn Tyr Lys Thr Thr Pro Pro Val Leu Asp Ser Asp Gly Ser
   545                 550                 555                 560
   Phe Phe Leu Tyr Ser Lys Leu Thr Val Asp Lys Ser Arg Trp Gln Gln
                   565                 570                 575
   Gly Asn Val Phe Ser Cys Ser Val Met His Glu Ala Leu His Asn His
               580                 585                 590
   Tyr Thr Gln Lys Ser Leu Ser Leu Ser Pro Gly Lys
           595                 600
<210> 15
   <211> 1812
   <212> DNA
   <213> Artificial
<220>
   <223> Human CTLA4 ECD -linker- anti-IL-17A mAb hc-B encoding nucleotide
   sequence
<400> 15
   gctatgcacg tcgctcagcc agcagtggtc ctggcatcca gcagaggcat cgccagtttc 60
   gtgtgcgagt acgcttcacc cggaaaggca acagaagtca gagtgactgt cctgcgacag 120
   gcagacagcc aggtgaccga ggtctgcgcc gctacatata tgatgggcaa cgaactgaca 180
   tttctggacg attctatctg tactggcacc tctagtggaa accaagtgaa tctgactatc 240
   cagggcctgc gcgctatgga taccggactg tacatttgca aggtggagct gatgtatccc 300
   cctccatact atctggggat tggtaatggc acccagatct acgtgattga cccagagccc 360
   tgtcctgact ctgatggagg tggaggatct ggaggtggag gaagcggggg tggcggatcc 420
   ggaggtggag gaagcggggg tggcggatct caggtccagc tccaagagtc cggccctggc 480
   ctggtcaagc ctagcgagac cctgtccctc acatgcaccg tgagcggctt tagcctgccc 540
   tcccattccg tgtcctggat taggcagccc cccggcaaag gactggagtg gatcggcatc 600
   atctggaacc agggcggaac cgactacaat agcgccttca agagccgggt gaccatctcc 660
   gtggacacca gcaagaacca gtttagcctg aagctgtcca gcgtcacagc cgccgacaca 720
   gctgtctact actgcgcccg gaacgcctac atcacagact actactacga gaactacttc 780
   atggacgcct ggggacaggg cacactggtg accgtgtcct ccgcctccac caaaggacct 840
   agcgtgtttc ccctcgcccc cagcagcaaa tccacctccg gaggaaccgc tgccctggga 900
   tgtctggtca aggactactt ccccgagccc gtgaccgtgt cctggaacag cggcgccctc 960
   acatccggcg tccacacatt tcccgccgtg ctgcaaagca gcggcctgta ctccctgagc 1020
   agcgtggtga ccgtgccttc ctccagcctg ggaacccaga cctacatctg caacgtgaac 1080
   cacaagccta gcaacaccaa ggtggacaag aaagtggagc ccaagagctg cgacaagacc 1140
   catacctgtc ccccttgccc tgcccctgaa ctgctgggag gccctagcgt gttcctgttt 1200
   ccacccaagc ccaaagacac actgatgatt tctcgcacac ccgaagtcac ttgtgtggtc 1260
   gtggacgtgt cccacgagga tcctgaagtc aagttcaact ggtacgtgga tggcgtcgag 1320
   gtgcataatg ctaagaccaa acccagagag gaacagtaca acagcaccta tcgcgtcgtg 1380
   tctgtcctga cagtgctgca ccaggattgg ctgaacggaa aggagtacaa gtgcaaagtg 1440
   agcaacaagg ccctgcccgc tcctatcgag aagaccattt ctaaggctaa aggccagcct 1500
   agagaaccac aggtgtatac actgcctcca agtcgcgacg agctgacaaa aaaccaggtc 1560
   tccctgactt gtctggtgaa gggattctac cctagcgata tcgcagtgga gtgggaatct 1620
   aatgggcagc cagaaaacaa ttataagacc acaccccctg tgctggactc agatggttcc 1680
   ttctttctgt acagtaaact gaccgtggac aagtccaggt ggcagcaggg gaacgtcttt 1740
   tcctgcagcg tgatgcatga ggccctgcac aatcattaca cacagaaatc tctgagtctg 1800
   tcaccaggaa ag 1812
<210> 16
   <211> 660
   <212> DNA
   <213> Artificial
<220>
   <223> anti-IL-17A mAb lc-B encoding nucleotide sequence
<400> 16
   gacatagtca tgacccagag ccccctgtcc ctgcctgtga cacctggaga gcccgcctcc 60
   atctcctgta agagctccca aagcctgctg ttcagcgaga accagaagaa ctacctggcc 120
   tggtatctgc aaaagcctgg ccagtccccc cagctcctca tctactggac aagcacccgg 180
   caaagcggcg tccctgacag gttctccgga agcggctccg gaaccgactt caccctgaaa 240
   ataagccggg tggaggctga ggacgtggga gtgtactact gccagcagtc ctactacacc 300
   ccctacacct tcggccaggg caccaaagtc gagatcaagc ggaccgtcgc cgctccctcc 360
   gtgttcatct ttccccccag cgatgagcag ctgaagtccg gcaccgctag cgtcgtgtgc 420
   ctcctgaaca acttctaccc ccgggaggcc aaggtccagt ggaaggtcga caacgccctc 480
   cagtccggca acagccaaga gagcgtgaca gagcaggata gcaaggactc cacctacagc 540
   ctgagcagca ccctgaccct cagcaaggcc gactacgaga agcacaaggt ctacgcttgc 600
   gaagtgaccc atcagggact cagctccccc gtcaccaaga gcttcaaccg gggcgagtgc 660
<210> 17
   <211> 32
   <212> DNA
   <213> Artificial
<220>
   <223> Primer
<400> 17
   ccggaattct cactttcctg gtgacagact ca 32
<210> 18
   <211> 28
   <212> DNA
   <213> Artificial
<220>
   <223> Primer
<400> 18
   ccggaattct cagcactcgc cccggttg 28
<210> 19
   <211> 364
   <212> PRT
   <213> Artificial
<220>
   <223> CTLA4 ECD -linker- anti-IL-17A mAb lc
<400> 19
   Ala Met His Val Ala Gln Pro Ala Val Val Leu Ala Ser Ser Arg Gly
   1               5                   10                  15
   Ile Ala Ser Phe Val Cys Glu Tyr Ala Ser Pro Gly Lys Ala Thr Glu
               20                  25                  30
   Val Arg Val Thr Val Leu Arg Gln Ala Asp Ser Gln Val Thr Glu Val
           35                  40                  45
   Cys Ala Ala Thr Tyr Met Met Gly Asn Glu Leu Thr Phe Leu Asp Asp
       50                  55                  60
   Ser Ile Cys Thr Gly Thr Ser Ser Gly Asn Gln Val Asn Leu Thr Ile
   65                  70                  75                  80
   Gln Gly Leu Arg Ala Met Asp Thr Gly Leu Tyr Ile Cys Lys Val Glu
                   85                  90                  95
   Leu Met Tyr Pro Pro Pro Tyr Tyr Leu Gly Ile Gly Asn Gly Thr Gln
               100                 105                 110
   Ile Tyr Val Ile Asp Pro Glu Pro Cys Pro Asp Ser Asp Gly Gly Gly
           115                 120                 125
   Gly Ser Gly Gly Gly Gly Ser Gly Gly Gly Gly Ser Gly Gly Gly Gly
       130                 135                 140
   Ser Gly Gly Gly Gly Ser Asp Ile Gln Met Thr Gln Ser Pro Ser Phe
   145                 150                 155                 160
   Leu Ser Ala Ser Val Gly Glu Arg Val Thr Leu Ser Cys Lys Ala Ser
                   165                 170                 175
   Gln Asn Ile Asn Lys Tyr Leu Asp Trp Phe Gln Gln Lys Leu Gly Glu
               180                 185                 190
   Ala Pro Lys Leu Leu Ile Tyr Asn Ala Asp Asn Leu His Thr Gly Ile
           195                 200                 205
   Pro Ser Arg Phe Ser Gly Ser Gly Ser Phe Ser Asp Phe Ile Leu Thr
       210                 215                 220
   Ile Ser Ser Leu Gln Pro Glu Asp Asp Ala Thr Tyr Phe Cys Leu Gln
   225                 230                 235                 240
   Arg Glu Ser Trp Pro Tyr Thr Phe Gly Ala Gly Thr Lys Leu Glu Leu
   245 250 255
   Lys Arg Ala Asp Ala Ala Pro Thr Val Ser Ile Phe Pro Pro Ser Ser
   260 265 270
   Glu Gln Leu Thr Ser Gly Gly Ala Ser Val Val Cys Phe Leu Asn Asn
   275 280 285
   Phe Tyr Pro Lys Asp Ile Asn Val Lys Trp Lys Ile Asp Gly Ser Glu
   290 295 300
   Arg Gln Asn Gly Val Leu Asn Ser Trp Thr Asp Gln Asp Ser Lys Asp
   305 310 315 320
   Ser Thr Tyr Ser Met Ser Ser Thr Leu Thr Leu Thr Lys Asp Glu Tyr
   325 330 335
   Glu Arg His Asn Ser Tyr Thr Cys Glu Ala Thr His Lys Thr Ser Thr
   340 345 350
   Ser Pro Ile Val Lys Ser Phe Asn Arg Asn Glu Cys
   355 360
<210> 20
   <211> 602
   <212> PRT
   <213> Artificial
<220>
   <223> anti-IL-17A mAb hc-A-linker-CTLA4 ECD
<400> 20
   Gln Val Gln Leu Lys Glu Ser Gly Pro Gly Leu Val Gln Pro Ser Gln
   1 5 10 15
   Thr Leu Ser Leu Thr Cys Thr Val Ser Gly Phe Ser Leu Thr Asn Tyr
   20 25 30
   Tyr Val His Trp Val Arg Gln Pro Pro Gly Lys Gly Leu Glu Trp Met
   35 40 45
   Gly Gly Val Trp Asn Asp Gly Asp Thr Ser Tyr Asn Ser Val Leu Arg
   50 55 60
   Ser Arg Leu Ser Ile Thr Arg Asp Thr Ser Lys Ser Gln Val Leu Leu
   65 70 75 80
   Lys Met Ser Ser Leu Gln Thr Glu Asp Thr Ala Thr Tyr Tyr Cys Ala
                   85                  90                  95
   Arg Glu Gly Arg Glu Gly Phe Val Gly Tyr Tyr Val Met Asp Ala Trp
               100                 105                 110
   Gly Pro Gly Ala Ser Val Thr Val Ser Ser Ala Lys Thr Thr Ala Pro
           115                 120                 125
   Ser Val Tyr Pro Leu Ala Pro Val Cys Gly Asp Thr Thr Gly Ser Ser
       130                 135                 140
   Val Thr Leu Gly Cys Leu Val Lys Gly Tyr Phe Pro Glu Pro Val Thr
   145                 150                 155                 160
   Leu Thr Trp Asn Ser Gly Ser Leu Ser Ser Gly Val His Thr Phe Pro
                   165                 170                 175
   Ala Val Leu Gln Ser Asp Leu Tyr Thr Leu Ser Ser Ser Val Thr Val
               180                 185                 190
   Thr Ser Ser Thr Trp Pro Ser Gln Ser Ile Thr Cys Asn Val Ala His
           195                 200                 205
   Pro Ala Ser Ser Thr Lys Val Asp Lys Lys Ile Glu Pro Arg Gly Pro
       210                 215                 220
   Thr Ile Lys Pro Cys Pro Pro Cys Lys Cys Pro Ala Pro Asn Leu Leu
   225                 230                 235                 240
   Gly Gly Pro Ser Val Phe Ile Phe Pro Pro Lys Ile Lys Asp Val Leu
                   245                 250                 255
   Met Ile Ser Leu Ser Pro Ile Val Thr Cys Val Val Val Asp Val Ser
               260                 265                 270
   Glu Asp Asp Pro Asp Val Gln Ile Ser Trp Phe Val Asn Asn Val Glu
           275                 280                 285
   Val His Thr Ala Gln Thr Gln Thr His Arg Glu Asp Tyr Asn Ser Thr
       290                 295                 300
   Leu Arg Val Val Ser Ala Leu Pro Ile Gln His Gln Asp Trp Met Ser
   305                 310                 315                 320
   Gly Lys Glu Phe Lys Cys Lys Val Asn Asn Lys Asp Leu Pro Ala Pro
                   325                 330                 335
   Ile Glu Arg Thr Ile Ser Lys Pro Lys Gly Ser Val Arg Ala Pro Gln
   340 345 350
   Val Tyr Val Leu Pro Pro Pro Glu Glu Glu Met Thr Lys Lys Gln Val
   355 360 365
   Thr Leu Thr Cys Met Val Thr Asp Phe Met Pro Glu Asp Ile Tyr Val
   370 375 380
   Glu Trp Thr Asn Asn Gly Lys Thr Glu Leu Asn Tyr Lys Asn Thr Glu
   385 390 395 400
   Pro Val Leu Asp Ser Asp Gly Ser Tyr Phe Met Tyr Ser Lys Leu Arg
   405 410 415
   Val Glu Lys Lys Asn Trp Val Glu Arg Asn Ser Tyr Ser Cys Ser Val
   420 425 430
   Val His Glu Gly Leu His Asn His His Thr Thr Lys Ser Phe Ser Arg
   435 440 445
   Thr Pro Gly Lys Gly Gly Gly Gly Ser Gly Gly Gly Gly Ser Gly Gly
   450 455 460
   Gly Gly Ser Gly Gly Gly Gly Ser Gly Gly Gly Gly Ser Ala Met His
   465 470 475 480
   Val Ala Gln Pro Ala Val Val Leu Ala Ser Ser Arg Gly Ile Ala Ser
   485 490 495
   Phe Val Cys Glu Tyr Ala Ser Pro Gly Lys Ala Thr Glu Val Arg Val
   500 505 510
   Thr Val Leu Arg Gln Ala Asp Ser Gln Val Thr Glu Val Cys Ala Ala
   515 520 525
   Thr Tyr Met Met Gly Asn Glu Leu Thr Phe Leu Asp Asp Ser Ile Cys
   530 535 540
   Thr Gly Thr Ser Ser Gly Asn Gln Val Asn Leu Thr Ile Gln Gly Leu
   545 550 555 560
   Arg Ala Met Asp Thr Gly Leu Tyr Ile Cys Lys Val Glu Leu Met Tyr
   565 570 575
   Pro Pro Pro Tyr Tyr Leu Gly Ile Gly Asn Gly Thr Gln Ile Tyr Val
   580 585 590
   Ile Asp Pro Glu Pro Cys Pro Asp Ser Asp
   595 600
<210> 21
   <211> 364
   <212> PRT
   <213> Artificial
<220>
   <223> anti-IL-17A mAb lc-A-linker-CTLA4 ECD
<400> 21
   Asp Ile Gln Met Thr Gln Ser Pro Ser Phe Leu Ser Ala Ser Val Gly
   1 5 10 15
   Glu Arg Val Thr Leu Ser Cys Lys Ala Ser Gln Asn Ile Asn Lys Tyr
   20 25 30
   Leu Asp Trp Phe Gln Gln Lys Leu Gly Glu Ala Pro Lys Leu Leu Ile
   35 40 45
   Tyr Asn Ala Asp Asn Leu His Thr Gly Ile Pro Ser Arg Phe Ser Gly
   50 55 60
   Ser Gly Ser Phe Ser Asp Phe Ile Leu Thr Ile Ser Ser Leu Gln Pro
   65 70 75 80
   Glu Asp Asp Ala Thr Tyr Phe Cys Leu Gln Arg Glu Ser Trp Pro Tyr
   85 90 95
   Thr Phe Gly Ala Gly Thr Lys Leu Glu Leu Lys Arg Ala Asp Ala Ala
   100 105 110
   Pro Thr Val Ser Ile Phe Pro Pro Ser Ser Glu Gln Leu Thr Ser Gly
   115 120 125
   Gly Ala Ser Val Val Cys Phe Leu Asn Asn Phe Tyr Pro Lys Asp Ile
   130 135 140
   Asn Val Lys Trp Lys Ile Asp Gly Ser Glu Arg Gln Asn Gly Val Leu
   145 150 155 160
   Asn Ser Trp Thr Asp Gln Asp Ser Lys Asp Ser Thr Tyr Ser Met Ser
   165 170 175
   Ser Thr Leu Thr Leu Thr Lys Asp Glu Tyr Glu Arg His Asn Ser Tyr
   180 185 190
   Thr Cys Glu Ala Thr His Lys Thr Ser Thr Ser Pro Ile Val Lys Ser
           195                 200                 205
   Phe Asn Arg Asn Glu Cys Gly Gly Gly Gly Ser Gly Gly Gly Gly Ser
       210                 215                 220
   Gly Gly Gly Gly Ser Gly Gly Gly Gly Ser Gly Gly Gly Gly Ser Ala
   225                 230                 235                 240
   Met His Val Ala Gln Pro Ala Val Val Leu Ala Ser Ser Arg Gly Ile
                   245                 250                 255
   Ala Ser Phe Val Cys Glu Tyr Ala Ser Pro Gly Lys Ala Thr Glu Val
               260                 265                 270
   Arg Val Thr Val Leu Arg Gln Ala Asp Ser Gln Val Thr Glu Val Cys
           275                 280                 285
   Ala Ala Thr Tyr Met Met Gly Asn Glu Leu Thr Phe Leu Asp Asp Ser
       290                 295                 300
   Ile Cys Thr Gly Thr Ser Ser Gly Asn Gln Val Asn Leu Thr Ile Gln
   305                 310                 315                 320
   Gly Leu Arg Ala Met Asp Thr Gly Leu Tyr Ile Cys Lys Val Glu Leu
                   325                 330                 335
   Met Tyr Pro Pro Pro Tyr Tyr Leu Gly Ile Gly Asn Gly Thr Gln Ile
               340                 345                 350
   Tyr Val Ile Asp Pro Glu Pro Cys Pro Asp Ser Asp
           355                 360
<210> 22
   <211> 330
   <212> PRT
   <213> Artificial
<220>
   <223> Human IgG1 heavy chain constant region
<400> 22
<210> 23
   <211> 106
   <212> PRT
   <213> Artificial
<220>
   <223> Human IgG1 light chain constant region
<400> 23
   Thr Val Ala Ala Pro Ser Val Phe Ile Phe Pro Pro Ser Asp Glu Gln
   1 5 10 15
   Leu Lys Ser Gly Thr Ala Ser Val Val Cys Leu Leu Asn Asn Phe Tyr
   20 25 30
   Pro Arg Glu Ala Lys Val Gln Trp Lys Val Asp Asn Ala Leu Gln Ser
   35 40 45
   Gly Asn Ser Gln Glu Ser Val Thr Glu Gln Asp Ser Lys Asp Ser Thr
   50 55 60
   Tyr Ser Leu Ser Ser Thr Leu Thr Leu Ser Lys Ala Asp Tyr Glu Lys
   65 70 75 80
   His Lys Val Tyr Ala Cys Glu Val Thr His Gln Gly Leu Ser Ser Pro
   85 90 95
   Val Thr Lys Ser Phe Asn Arg Gly Glu Cys
   100 105
<210> 24
   <211> 602
   <212> PRT
   <213> Artificial
<220>
   <223> The chimera CTLA4 ECD-linker- anti-IL-17A mAb hc of the bifunctional
   fusion protein A
<400> 24
   Ala Met His Val Ala Gln Pro Ala Val Val Leu Ala Ser Ser Arg Gly
   1 5 10 15
   Ile Ala Ser Phe Val Cys Glu Tyr Ala Ser Pro Gly Lys Ala Thr Glu
   20 25 30
   Val Arg Val Thr Val Leu Arg Gln Ala Asp Ser Gln Val Thr Glu Val
   35 40 45
   Cys Ala Ala Thr Tyr Met Met Gly Asn Glu Leu Thr Phe Leu Asp Asp
   50 55 60
   Ser Ile Cys Thr Gly Thr Ser Ser Gly Asn Gln Val Asn Leu Thr Ile
   65 70 75 80
   Gln Gly Leu Arg Ala Met Asp Thr Gly Leu Tyr Ile Cys Lys Val Glu
   85 90 95
   Leu Met Tyr Pro Pro Pro Tyr Tyr Leu Gly Ile Gly Asn Gly Thr Gln
   100 105 110
   Ile Tyr Val Ile Asp Pro Glu Pro Cys Pro Asp Ser Asp Gly Gly Gly
   115 120 125
   Gly Ser Gly Gly Gly Gly Ser Gly Gly Gly Gly Ser Gly Gly Gly Gly
   130 135 140
   Ser Gly Gly Gly Gly Ser Gln Val Gln Leu Lys Glu Ser Gly Pro Gly
   145 150 155 160
   Leu Val Gln Pro Ser Gln Thr Leu Ser Leu Thr Cys Thr Val Ser Gly
   165 170 175
   Phe Ser Leu Thr Asn Tyr Tyr Val His Trp Val Arg Gln Pro Pro Gly
   180 185 190
   Lys Gly Leu Glu Trp Met Gly Gly Val Trp Asn Asp Gly Asp Thr Ser
   195 200 205
   Tyr Asn Ser Val Leu Arg Ser Arg Leu Ser Ile Thr Arg Asp Thr Ser
   210 215 220
   Lys Ser Gln Val Leu Leu Lys Met Ser Ser Leu Gln Thr Glu Asp Thr
   225 230 235 240
   Ala Thr Tyr Tyr Cys Ala Arg Glu Gly Arg Glu Gly Phe Val Gly Tyr
   245 250 255
   Tyr Val Met Asp Ala Trp Gly Pro Gly Ala Ser Val Thr Val Ser Ser
               260                 265                 270
   Ala Ser Thr Lys Gly Pro Ser Val Phe Pro Leu Ala Pro Ser Ser Lys
           275                 280                 285
   Ser Thr Ser Gly Gly Thr Ala Ala Leu Gly Cys Leu Val Lys Asp Tyr
       290                 295                 300
   Phe Pro Glu Pro Val Thr Val Ser Trp Asn Ser Gly Ala Leu Thr Ser
   305                 310                 315                 320
   Gly Val His Thr Phe Pro Ala Val Leu Gln Ser Ser Gly Leu Tyr Ser
                   325                 330                 335
   Leu Ser Ser Val Val Thr Val Pro Ser Ser Ser Leu Gly Thr Gln Thr
               340                 345                 350
   Tyr Ile Cys Asn Val Asn His Lys Pro Ser Asn Thr Lys Val Asp Lys
           355                 360                 365
   Lys Val Glu Pro Lys Ser Cys Asp Lys Thr His Thr Cys Pro Pro Cys
       370                 375                 380
   Pro Ala Pro Glu Leu Leu Gly Gly Pro Ser Val Phe Leu Phe Pro Pro
   385                 390                 395                 400
   Lys Pro Lys Asp Thr Leu Met Ile Ser Arg Thr Pro Glu Val Thr Cys
                   405                 410                 415
   Val Val Val Asp Val Ser His Glu Asp Pro Glu Val Lys Phe Asn Trp
               420                 425                 430
   Tyr Val Asp Gly Val Glu Val His Asn Ala Lys Thr Lys Pro Arg Glu
           435                 440                 445
   Glu Gln Tyr Asn Ser Thr Tyr Arg Val Val Ser Val Leu Thr Val Leu
       450                 455                 460
   His Gln Asp Trp Leu Asn Gly Lys Glu Tyr Lys Cys Lys Val Ser Asn
   465                 470                 475                 480
   Lys Ala Leu Pro Ala Pro Ile Glu Lys Thr Ile Ser Lys Ala Lys Gly
                   485                 490                 495
   Gln Pro Arg Glu Pro Gln Val Tyr Thr Leu Pro Pro Ser Arg Asp Glu
               500                 505                 510
   Leu Thr Lys Asn Gln Val Ser Leu Thr Cys Leu Val Lys Gly Phe Tyr
           515                 520                 525
   Pro Ser Asp Ile Ala Val Glu Trp Glu Ser Asn Gly Gln Pro Glu Asn
       530                 535                 540
   Asn Tyr Lys Thr Thr Pro Pro Val Leu Asp Ser Asp Gly Ser Phe Phe
   545                 550                 555                 560
   Leu Tyr Ser Lys Leu Thr Val Asp Lys Ser Arg Trp Gln Gln Gly Asn
                   565                 570                 575
   Val Phe Ser Cys Ser Val Met His Glu Ala Leu His Asn His Tyr Thr
               580                 585                 590
   Gln Lys Ser Leu Ser Leu Ser Pro Gly Lys
           595                 600
<210> 25
   <211> 214
   <212> PRT
   <213> Artificial
<220>
   <223> The chimera anti-IL-17A mAb lc of the bifunctional fusion protein A
<400> 25
   Asp Ile Gln Met Thr Gln Ser Pro Ser Phe Leu Ser Ala Ser Val Gly
   1               5                   10                  15
   Glu Arg Val Thr Leu Ser Cys Lys Ala Ser Gln Asn Ile Asn Lys Tyr
               20                  25                  30
   Leu Asp Trp Phe Gln Gln Lys Leu Gly Glu Ala Pro Lys Leu Leu Ile
           35                  40                  45
   Tyr Asn Ala Asp Asn Leu His Thr Gly Ile Pro Ser Arg Phe Ser Gly
       50                  55                  60
   Ser Gly Ser Phe Ser Asp Phe Ile Leu Thr Ile Ser Ser Leu Gln Pro
   65                  70                  75                  80
   Glu Asp Asp Ala Thr Tyr Phe Cys Leu Gln Arg Glu Ser Trp Pro Tyr
                   85                  90                  95
   Thr Phe Gly Ala Gly Thr Lys Leu Glu Leu Lys Arg Thr Val Ala Ala
               100                 105                 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 26
   <211> 602
   <212> PRT
   <213> Artificial
<220>
   <223> The humanized analogue CTLA4 ECD-linker- anti-IL-17A mAb hc of the
   bifunctional fusion protein A
<400> 26
   Ala Met His Val Ala Gln Pro Ala Val Val Leu Ala Ser Ser Arg Gly
   1 5 10 15
   Ile Ala Ser Phe Val Cys Glu Tyr Ala Ser Pro Gly Lys Ala Thr Glu
   20 25 30
   Val Arg Val Thr Val Leu Arg Gln Ala Asp Ser Gln Val Thr Glu Val
   35 40 45
   Cys Ala Ala Thr Tyr Met Met Gly Asn Glu Leu Thr Phe Leu Asp Asp
   50 55 60
   Ser Ile Cys Thr Gly Thr Ser Ser Gly Asn Gln Val Asn Leu Thr Ile
   65 70 75 80
   Gln Gly Leu Arg Ala Met Asp Thr Gly Leu Tyr Ile Cys Lys Val Glu
   85 90 95
   Leu Met Tyr Pro Pro Pro Tyr Tyr Leu Gly Ile Gly Asn Gly Thr Gln
               100                 105                 110
   Ile Tyr Val Ile Asp Pro Glu Pro Cys Pro Asp Ser Asp Gly Gly Gly
           115                 120                 125
   Gly Ser Gly Gly Gly Gly Ser Gly Gly Gly Gly Ser Gly Gly Gly Gly
       130                 135                 140
   Ser Gly Gly Gly Gly Ser Glu Val Gln Leu Val Glu Ser Gly Gly Gly
   145                 150                 155                 160
   Leu Val Gln Pro Gly Arg Ser Leu Arg Leu Ser Cys Ala Ala Ser Gly
                   165                 170                 175
   Phe Ser Leu Thr Asn Tyr Ala Met His Trp Val Arg Gln Ala Pro Gly
               180                 185                 190
   Lys Gly Leu Glu Trp Val Ser Ala Ile Trp Asn Asp Gly Asp Ile Asp
           195                 200                 205
   Tyr Ala Asp Ser Val Glu Gly Arg Phe Thr Ile Ser Arg Asp Asn Ala
       210                 215                 220
   Lys Asn Ser Leu Tyr Leu Gln Met Asn Ser Leu Arg Ala Glu Asp Thr
   225                 230                 235                 240
   Ala Val Tyr Tyr Cys Ala Lys Glu Gly Arg Glu Gly Phe Val Gly Tyr
                   245                 250                 255
   Tyr Val Met Asp Ala Trp Gly Gln Gly Thr Leu Val Thr Val Ser Ser
               260                 265                 270
   Ala Ser Thr Lys Gly Pro Ser Val Phe Pro Leu Ala Pro Ser Ser Lys
           275                 280                 285
   Ser Thr Ser Gly Gly Thr Ala Ala Leu Gly Cys Leu Val Lys Asp Tyr
       290                 295                 300
   Phe Pro Glu Pro Val Thr Val Ser Trp Asn Ser Gly Ala Leu Thr Ser
   305                 310                 315                 320
   Gly Val His Thr Phe Pro Ala Val Leu Gln Ser Ser Gly Leu Tyr Ser
                   325                 330                 335
   Leu Ser Ser Val Val Thr Val Pro Ser Ser Ser Leu Gly Thr Gln Thr
               340                 345                 350
   Tyr Ile Cys Asn Val Asn His Lys Pro Ser Asn Thr Lys Val Asp Lys
           355                 360                 365
   Lys Val Glu Pro Lys Ser Cys Asp Lys Thr His Thr Cys Pro Pro Cys
       370                 375                 380
   Pro Ala Pro Glu Leu Leu Gly Gly Pro Ser Val Phe Leu Phe Pro Pro
   385                 390                 395                 400
   Lys Pro Lys Asp Thr Leu Met Ile Ser Arg Thr Pro Glu Val Thr Cys
                   405                 410                 415
   Val Val Val Asp Val Ser His Glu Asp Pro Glu Val Lys Phe Asn Trp
               420                 425                 430
   Tyr Val Asp Gly Val Glu Val His Asn Ala Lys Thr Lys Pro Arg Glu
           435                 440                 445
   Glu Gln Tyr Asn Ser Thr Tyr Arg Val Val Ser Val Leu Thr Val Leu
       450                 455                 460
   His Gln Asp Trp Leu Asn Gly Lys Glu Tyr Lys Cys Lys Val Ser Asn
   465                 470                 475                 480
   Lys Ala Leu Pro Ala Pro Ile Glu Lys Thr Ile Ser Lys Ala Lys Gly
                   485                 490                 495
   Gln Pro Arg Glu Pro Gln Val Tyr Thr Leu Pro Pro Ser Arg Asp Glu
               500                 505                 510
   Leu Thr Lys Asn Gln Val Ser Leu Thr Cys Leu Val Lys Gly Phe Tyr
           515                 520                 525
   Pro Ser Asp Ile Ala Val Glu Trp Glu Ser Asn Gly Gln Pro Glu Asn
       530                 535                 540
   Asn Tyr Lys Thr Thr Pro Pro Val Leu Asp Ser Asp Gly Ser Phe Phe
   545                 550                 555                 560
   Leu Tyr Ser Lys Leu Thr Val Asp Lys Ser Arg Trp Gln Gln Gly Asn
                   565                 570                 575
   Val Phe Ser Cys Ser Val Met His Glu Ala Leu His Asn His Tyr Thr
               580                 585                 590
   Gln Lys Ser Leu Ser Leu Ser Pro Gly Lys
           595                 600
<210> 27
   <211> 214
   <212> PRT
   <213> Artificial
<220>
   <223> The humanized analogue anti-IL-17A mAb lc of the bifunction fusion
   protein A
<400> 27
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Ile Asn Lys Tyr
               20                  25                  30
   Leu Asp Trp Tyr Gln Gln Lys Pro Gly Lys Ala Pro Lys Leu Leu Ile
           35                  40                  45
   Tyr Asn Ala Asp Asn Leu His Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Leu Gln Arg Glu Ser Trp Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
   210
<210> 28
   <211> 452
   <212> PRT
   <213> Artificial
<220>
   <223> The humanized analogue anti-IL-17A mAb hc of the bifunctional fusion
   protein A
<400> 28
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Ser Leu Thr Asn Tyr
               20                  25                  30
   Ala Met His Trp Val Arg Gln Ala Pro Gly Lys Gly Leu Glu Trp Val
           35                  40                  45
   Ser Ala Ile Trp Asn Asp Gly Asp Ile Asp Tyr Ala Asp Ser Val Glu
       50                  55                  60
   Gly Arg Phe Thr Ile Ser Arg Asp Asn Ala Lys Asn Ser Leu Tyr Leu
   65                  70                  75                  80
   Gln Met Asn Ser Leu Arg Ala Glu Asp Thr Ala Val Tyr Tyr Cys Ala
                   85                  90                  95
   Lys Glu Gly Arg Glu Gly Phe Val Gly Tyr Tyr Val Met Asp Ala Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro Lys Ser
       210                 215                 220
   Cys Asp Lys Thr His Thr Cys Pro Pro Cys Pro Ala Pro Glu Leu Leu
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
       290                 295                 300
   Tyr Arg Val Val Ser Val Leu Thr Val Leu His Gln Asp Trp Leu Asn
   305                 310                 315                 320
   Gly Lys Glu Tyr Lys Cys Lys Val Ser Asn Lys Ala Leu Pro Ala Pro
                   325                 330                 335
   Ile Glu Lys Thr Ile Ser Lys Ala Lys Gly Gln Pro Arg Glu Pro Gln
               340                 345                 350
   Val Tyr Thr Leu Pro Pro Ser Arg Asp Glu Leu Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Val Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
   435 440 445
   Ser Pro Gly Lys
   450